(19) Europäisches Patentamt / European Patent Office / Office européen des brevets

(11) EP 2 794 572 B1

(12) # FASCICULE DE BREVET EUROPEEN

(45) Date de publication et mention de la délivrance du brevet: **03.08.2016 Bulletin 2016/31**

(21) Numéro de dépôt: **12812261.1**

(22) Date de dépôt: **21.12.2012**

(51) Int Cl.: ***C07D 219/06*** *(2006.01)* ***A61K 31/435*** *(2006.01)* ***A61P 17/00*** *(2006.01)*

(86) Numéro de dépôt international: **PCT/EP2012/076681**

(87) Numéro de publication internationale: **WO 2013/093019 (27.06.2013 Gazette 2013/26)**

(54) **DERIVÉS D'ACRIDINEDIONE POUR LE TRAITEMENT DES TROUBLES DE LA PIGMENTATION ET DU VIEILLISSEMENT CUTANÉ**

ACRIDINDIONDERIVATE ZUR BEHANDLUNG VON PIGMENTIERUNGSSTÖRUNGEN UND HAUTALTERUNG

ACRIDINEDIONE DERIVATIVES FOR TREATING PIGMENTATION DISORDERS AND AGEING OF THE SKIN

(84) Etats contractants désignés: **AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.12.2011 FR 1162204**

(43) Date de publication de la demande: **29.10.2014 Bulletin 2014/44**

(73) Titulaire: **Pierre Fabre Dermo-Cosmétique**
**92100 Boulogne-Billancourt (FR)**

(72) Inventeurs:
- **POIGNY, Stéphane**
  **F-31600 Saubens (FR)**
- **BELAUBRE, Françoise**
  **F-31270 Villeneuve Tolosane (FR)**

(74) Mandataire: **Regimbeau**
**20, rue de Chazelles**
**75847 Paris Cedex 17 (FR)**

(56) Documents cités:
- **ASHOKKUMAR, PICHANDI ET AL: "Specific Ca2+ Fluorescent Sensor: Signaling by Conformationally Induced PET Suppression in a Bichromophoric Acridinedione", EUROPEAN JOURNAL OF ORGANIC CHEMISTRY , (34), 5941-5947, S5941/1-S5941/19 CODEN: EJOCFK; ISSN: 1434-193X, 2009, XP002673808,**
- **THIMMAPPA H MANJASHETTY ET AL: "Microwave assisted one-pot synthesis of highly potent novel isoniazid analogues", BIOORGANIC & MEDICINAL CHEMISTRY LETTERS, PERGAMON, ELSEVIER SCIENCE, GB, vol. 21, no. 7, 28 janvier 2011 (2011-01-28), pages 2125-2128, XP028162350, ISSN: 0960-894X, DOI: 10.1016/J.BMCL.2011.01.122 [extrait le 2011-02-02]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne des dérivés d'acridinedione ainsi que les compositions topiques les contenant, leur procédé de préparation et leurs utilisations, notamment en tant que médicament ou en tant que principe actif cosmétique.

L'Etat de la technique antérieure peut être illustré par Thimmappa H. Manjashetty et al. (« Microwave assisted one-pot synthesis of highly potent novel isoniazid analogues », Bioorg. & Med. Chem. Lett., Pergamon, Elsevier Science, GB, vol.21, n°7, 28 janvier 2011, pp 2125-28) qui décrit une nouvele série d'analogues d'isoniazide et en particulier le composé N-[2,3,4,5,6,7,8,9-octahydro-9-(4-hydroxy-3-methoxyphényl)-3,3,6,6-tetraméthyl-1,8-dioxo-10(1H)-acridinyl]-4-pyridinecarboxamide utile comme médicament dans le traitement de la tuberculose.

Ces composés se distinguent de l'objet de la présente invention à la fois en termes de structure et d'activité.

Ainsi la présente invention a pour objet un composé de la formule générale (I) suivante :

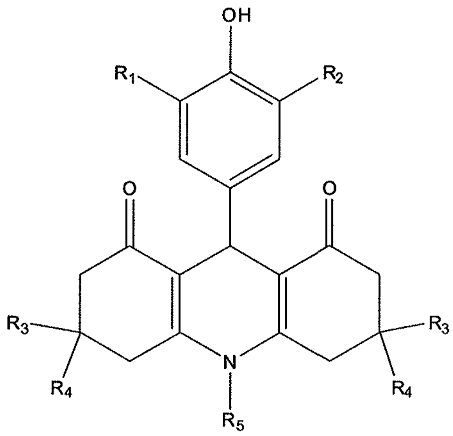

(I)

dans laquelle :

- $R_1$ et $R_2$ représentent simultanément ou indépendamment: H, OH, $OCH_3$ ou un radical alkyle en $C_1$-$C_5$,
- $R_3$ et $R_4$ représentent simultanément $CH_3$, 15ou $R_3$ représente H, et $R_4$ représente $CH_3$, $CH_2CH_3$ ou un radical isopropyle, ou un radical phényle,
- $R_5$ représente : un radical alkyle en $C_4$-$C_{24}$ ou un radical 3-phénylpropanyle ou 2,2-diphényléthanyle.

Par "radical alkyle", on entend au sens de la présente invention une chaîne hydrocarbonée aliphatique linéaire ou ramifiée saturée et comprenant le nombre d'atomes de carbone spécifié. On peut par exemple citer, le méthyle, l'éthyle et le propyle. Le radical alkyle peut notamment représenter la chaîne hydrocarbonée d'un acide gras saturé de $C_1$-$C_{24}$ et en particulier de $C_{10}$-$C_{24}$.

Les acides gras saturés peuvent être l'acide caprique (10:0), l'acide undécylique (11:0), l'acide laurique (12:0), l'acide tridécylique (13:0), l'acide myristique (14:0), l'acide pentadécylique (15:0), l'acide palmitique (16:0), l'acide margarique (17:0), l'acide stéarique (18:0), l'acide nonadécylique (19:0), l'acide arachidique (20:0), l'acide hénéicosanoïque (21:0), l'acide béhénique (22:0), l'acide tricosanoïque (23:0), l'acide lignocérique (24:0). En particulier, les acides gras saturés peuvent être l'acide palmitique et l'acide stéarique.

Selon un mode de réalisation particulier de l'invention, les composés de formule générale (I) sont ceux pour lesquels $R_1$ représente H et $R_2$ représente $OCH_3$.

De préférence, les composés de formule générale (I) sont ceux pour lesquels $R_1$ représente H, $R_2$ représente $OCH_3$ ; et $R_3$ et $R_4$ représentent simultanément $CH_3$.

Les composés de formule générale (I) peuvent être choisis parmi la liste de composés suivants :

- 10-dodécyl-9-(4-hydroxy-3-méthoxyphényl)-3,3,6,6-tétraméthyl-3,4,6,7,9,10-hexahydroacridine-1,8(2H,5H)-dione (exemple 1) ;
- 10-décyl-9-(3,4-dihydroxyphényl)-3,3,6,6-tétraméthyl-3,4,6,7,9,10-hexahydroacridine-1,8(2H,5H)-dione (exemple 3) ;
- 9-(3,4-dihydroxyphényl)-10-méthyl-3,6-diphényl-3,4,6,7,9,10-hexahydroacridine-1,8(2H,5H)-dione (exemple 5) ;
- 10-décyl-9-(3,4-dihydroxyphéyl)-3,6-diphéyl-3,4,6,7,9,10-hexahydroacridine-1,8(2H,5H)-dione (exemple 6) ;
- 10-décyl-9-(4-hydroxy-3,5-diméthoxyphényl)-3,3,6,6-tétraméthyl-3,4,6,7,9,10-hexahydroacridine-1,8(2H,5H)-dione (exemple 7) ;

- 10-dodécyl-9-(3,5-di-tert-butyl-4-hydroxyphényl)-3,3,6,6-tétraméthyl-3,4,6,7,9,10-hexahydroacridine-1,8(2H,5H)-dione (exemple 8) ;
- 10-dodécyl-9-(4-hydroxy-3-méthoxyphényl)-3,6-diisopropyl-3,4,6,7,9,10-hexahydroacridine-1,8(2H,5H)-dione (exemple 9) ;
- 10-butyl-9-(4-hydroxy-3-méthoxyphényl)-3,3,6,6-tétraméthyl-3,4,6,7,9,10-hexahydroacridine-1,8(2H,5H)-dione (exemple 14) ;
- 9-(4-hydroxy-3-méthoxyphényl)-3,3,6,6-tétraméthyl-10-pentyl-3,4,6,7,9,10-hexahydroacridine-1,8(2H,5H)-dione (exemple 15) ;
- 10-hexyl-9-(4-hydroxy-3-méthoxyphényl)-3,3,6,6-tétraméthyl-3,4,6,7,9,10-hexahydroacridine-1,8(2H,5H)-dione (exemple 16) ;
- 10-heptyl-9-(4-hydroxy-3-méthoxyphényl)-3,3,6,6-tétraméthyl-3,4,6,7,9,10-hexahydroacridine-1,8(2H,5H)-dione (exemple 17) ;
- 9-(4-hydroxy-3-méthoxyphényl)-3,3,6,6-tétraméthyl-10-octyl-3,4,6,7,9,10-hexahydroacridine-1,8(2H,5H)-dione (exemple 18) ;
- 9-(4-hydroxy-3-méthoxyphényl)-3,3,6,6-tétraméthyl-10-nonyl-3,4,6,7,9,10-hexahydroacridine-1,8(2H,5H)-dione (exemple 19) ;
- 9-(4-hydroxy-3-méthoxyphényl)-3,3,6,6-tétraméthyl-10-undécyl-3,4,6,7,9,10-hexahydroacridine-1,8(2H,5H)-dione (exemple 20)
- 10-benzyl-9-(4-hydroxy-3-méthoxyphényl)-3,3,6,6-tétraméthyl-3,4,6,7,9,10-hexahydroacridine-1,8(2H,5H)-dione (exemple 21) ;
- 9-(4-hydroxy-3-méthoxyphényl)-3,3,6,6-tétraméthyl-10-(3-phénylpropyl)-3,4,6,7,9,10-hexahydroacridine-1,8(2H,5H)-dione (exemple 23) ;
- 10-(2,2-diphényléthyl)-9-(4-hydroxy-3-méthoxyphényl)-3,3,6,6-tétraméthyl-3,4,6,7,9,10-hexahydroacridine-1,8(2H,5H)-dione (exemple 24).

La présente invention s'étend également aux composés de formule générale (I') :

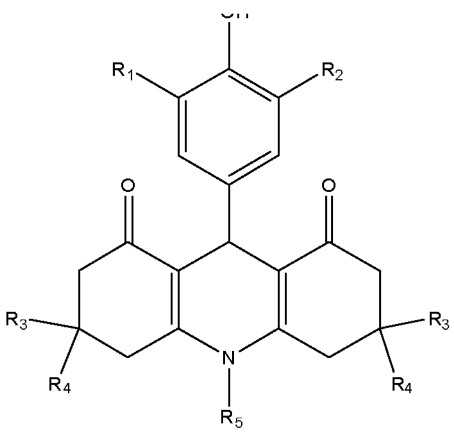

(I')

dans laquelle :

- $R_1$ et $R_2$ représentent simultanément ou indépendamment H, OH, $OCH_3$ ou un radical alkyle en $C_1$-$C_5$,
- $R_3$ et $R_4$ représentent simultanément $CH_3$,
  ou $R_3$ représente H et $R_4$ représente $CH_3$, $CH_2CH_3$, un radical isopropyle, ou un radical phényle,
- $R_5$ représente : H, un radical alkyle en $C_1$-$C_{24}$, un radical benzyle, un radical phénéthyle ou un radical 3-phénylpropanyle ou 2,2-diphényléthanyle, pour leur utilisation topique en tant que médicament.

L'invention concerne également les composés de formule générale (I') définie ci-dessus pour leur utilisation topique visant à modifier la pigmentation de la peau et/ou des cheveux et/ou des poils.

L'invention concerne également les composés de formule générale (I') définie ci-dessus qui, lorsqu'ils présentent un log P > 4, sont généralement utiles dans la dépigmentation de la peau et/ou des cheveux et/ou des poils.

L'invention concerne également les composés de formule générale (I') définie ci-dessus qui, lorsqu'ils présentent un log P < 3,5, sont généralement utiles dans la propigmentation de la peau et/ou des cheveux et/ou des poils.

LogP, aussi appelé *Log Kow,* est une mesure de la solubilité différentielle de composés chimiques dans deux solvants (coefficient de partage octanol/eau).

LogP est égal au logarithme du rapport des concentrations de la substance étudiée dans l'octanol et dans l'eau. LogP = Log($C_{oct}$/$C_{eau}$). Cette valeur permet d'appréhender le caractère hydrophile ou hydrophobe (lipophile) d'une molécule. En effet, si LogP est positif et très élevé, cela exprime le fait que la molécule considérée est bien plus soluble dans l'octanol que dans l'eau, ce qui reflète son caractère lipophile, et inversement. Une valeur de LogP = 0 signifie que la molécule se répartit de manière égale entre les deux phases et $C_{oct}$=$C_{eau}$ (C. Hansch, J. F. Quinlan, G. L. Lawrence, Linear free energy relationship between partition coefficients and the aqueous solubility of organic liquids, J. Org. Chem. , 33 (1968), 347-350 ; C. Hansch, A. Leo, Exploring QSAR - Fundamentals and Applications in Chemistry and Biology, American Chemical Society, Washington, 1995 ; J. Sangster, Octanol - Water Partition Coefficients: Fundamentals and Physical Chemistry, Wiley, Chichester, 1997 ; W. Jorgensen, E. Duffy, Advanced Drug Delivery Reviews, (54), 2002, 355-366).

L'invention concerne également les composés de formule générale (I') définie ci-dessus dans laquelle $R_1$ représente H, $R_2$ représente $OCH_3$, et $R_3$ et $R_4$ représentent simultanément $CH_3$ dans le traitement et/ou la prévention du vieillissement de la peau et le traitement et/ou la prévention des troubles de la pigmentation.

L'invention concerne également les composés de formule générale (I') définie ci-dessus dans laquelle $R_1$ représente H, $R_2$ représente $OCH_3$, et $R_3$ et $R_4$ représentent simultanément $CH_3$ :

- dans la dépigmentation de la peau et/ou des cheveux et/ou des poils généralement lorsque le composé présente un log P > 4 ;
- ou la propigmentation de la peau et/ou des cheveux et/ou des poils généralement lorsque le composé présente un log P < 3,5.

La présente invention concerne l'utilisation cosmétique desdits composés de formule générale (I') en tant que :

- principe actif anti-oxydant,
- principe actif dépigmentant généralement lorsque le composé présente un log P > 4 ;
- ou principe actif propigmentant généralement lorsque le composé présente un log P < 3,5.

L'activité dépigmentante, qui consiste de façon générale à réduire et/ou inhiber la production des mélanines responsables de la pigmentation, ou à réduire le transport de mélanines dans les dendrites, peut être déclinée en différents types d'actions au sens de la présente invention :

- réduire et/ou supprimer les taches de pigmentation, telles que les taches d'hyperpigmentation dues à un stress pro-inflammatoire (taches pigmentaires brunâtres UV induites par exemple) et les chloasmas, ou bien :
    - blanchir et/ou éclaircir la peau et/ou les poils et/ou les cheveux, préférentiellement afin :
        - ✓ d'unifier le teint, ce qui se caractérise par l'obtention d'un teint de peau uniforme, plus clair, plus transparent, plus lumineux. L'éclat du teint se trouve ainsi amélioré. Les avantages obtenus sont particulièrement intéressants pour les peaux sensibles quelle que soit leur nature (sèche, normale, grasse) et plus particulièrement pour les peaux sensibles ternes et sans éclat,

    et/ou :

        - ✓ de traiter certaines taches pigmentaires inesthétiques dues à une hyperpigmentation épidermique, telles que les taches de sénescence de la peau notamment. L'activité dépigmentante au sens de la présente invention se traduit alors par l'atténuation visible de l'intensité et de la taille des taches pigmentaires et/ou par l'empêchement de l'apparition de taches supplémentaires.

L'activité propigmentante consiste quant à elle à favoriser l'augmentation de la synthèse de mélanine dans les mélanocytes de l'épiderme ou du bulbe pileux :

- en vue de lui donner un aspect bronzé, ou de préparer la peau à une exposition au soleil ;
- mais aussi à des fins thérapeutiques pour repigmenter la peau dépigmentée, par exemple dans le cas du vitiligo, ou pour pigmenter les poils ou les cheveux, en particulier dans le traitement et la prévention de la canitie.

La présente invention concerne l'utilisation en tant que principe actif antioxydant, principe actif dépigmentant

ou principe actif propigmentant, des composés de formule générale (I') dans laquelle $R_1$ représente H, $R_2$ représente $OCH_3$, et $R_3$ et $R_4$ représentent simultanément $CH_3$.

La présente invention concerne également l'utilisation topique des compositions cosmétiques comprenant un composé de formule générale (I') dans le traitement et/ou à la prévention du vieillissement de la peau et le traitement et/ou la prévention des troubles de la pigmentation.

L'invention concerne également l'utilisation topique des compositions cosmétiques comprenant un composé de formule générale (I') définie ci-dessus et qui, lorsqu'ils présentent un log P > 4, sont généralement utiles dans la dépigmentation de la peau et/ou des cheveux et/ou des poils.

L'invention concerne également l'utilisation topique des compositions cosmétiques comprenant un composé de formule générale (I') définie ci-dessus qui, lorsqu'ils présentent un log P < 3,5, sont généralement utiles dans la propigmentation de la peau et/ou des cheveux et/ou des poils.

Les composés de formule générale (I') peuvent être choisis parmi la liste de composés suivants :

- 10-dodécyl-9-(4-hydroxy-3-méthoxyphényl)-3,3,6,6-tétraméthyl-3,4,6,7,9,10-hexahydroacridine-1,8(2H,5H)-dione (exemple 1) ;
- 10-décyl-9-(3,4-dihydroxyphényl)-3,3,6,6-tétraméthyl-3,4,6,7,9,10-hexahydroacridine-1,8(2H,5H)-dione (exemple 3) ;
- 9-(3,4-dihydroxyphényl)-10-méthyl-3,6-diphényl-3,4,6,7,9,10-hexahydroacridine-1,8(2H,5H)-dione (exemple 5) ;
- 10-décyl-9-(3,4-dihydroxyphéyl)-3,6-diphéyl-3,4,6,7,9,10-hexahydroacridine-1,8(2H,5H)-dione (exemple 6) ;
- 10-décyl-9-(4-hydroxy-3,5-diméthoxyphényl)-3,3,6,6-tétraméthyl-3,4,6,7,9,10-hexahydroacridine-1,8(2H,5H)-dione (exemple 7) ;
- 10-dodécyl-9-(3,5-di-tert-butyl-4-hydroxyphényl)-3,3,6,6-tétraméthyl-3,4,6,7,9,10-hexahydroacridine-1,8(2H,5H)-dione (exemple 8) ;
- 10-dodécyl-9-(4-hydroxy-3-méthoxyphényl)-3,6-diisopropyl-3,4,6,7,9,10-hexahydroacridine-1,8(2H,5H)-dione (exemple 9) ;
- 10-butyl-9-(4-hydroxy-3-méthoxyphényl)-3,3,6,6-tétraméthyl-3,4,6,7,9,10-hexahydroacridine-1,8(2H,5H)-dione (exemple 14) ;
- 9-(4-hydroxy-3-méthoxyphényl)-3,3,6,6-tétraméthyl-10-pentyl-3,4,6,7,9,10-hexahydroacridine-1,8(2H,5H)-dione (exemple 15) ;
- 10-hexyl-9-(4-hydroxy-3-méthoxyphényl)-3,3,6,6-tétraméthyl-3,4,6,7,9,10-hexahydroacridine-1,8(2H,5H)-dione (exemple 16) ;
- 10-heptyl-9-(4-hydroxy-3-méthoxyphényl)-3,3,6,6-tétraméthyl-3,4,6,7,9,10-hexahydroacridine-1,8(2H,5H)-dione (exemple 17) ;
- 9-(4-hydroxy-3-méthoxyphényl)-3,3,6,6-tétraméthyl-10-octyl-3,4,6,7,9,10-hexahydroacridine-1,8(2H,5H)-dione (exemple 18) ;
- 9-(4-hydroxy-3-méthoxyphényl)-3,3,6,6-tétraméthyl-10-nonyl-3,4,6,7,9,10-hexahydroacridine-1,8(2H,5H)-dione (exemple 19) ;
- 9-(4-hydroxy-3-méthoxyphényl)-3,3,6,6-tétraméthyl-10-undécyl-3,4,6,7,9,10-hexahydroacridine-1,8(2H,5H)-dione (exemple 20) ;
- 10-benzyl-9-(4-hydroxy-3-méthoxyphényl)-3,3,6,6-tétraméthyl-3,4,6,7,9,10-hexahydroacridine-1,8(2H,5H)-dione (exemple 21) ;
- 9-(4-hydroxy-3-méthoxyphényl)-3,3,6,6-tétraméthyl-10-(3-phénylpropyl)-3,4,6,7,9,10-hexahydroacridine-1,8(2H,5H)-dione (exemple 23) ;
- 10-(2,2-diphényléthyl)-9-(4-hydroxy-3-méthoxyphényl)-3,3,6,6-tétraméthyl-3,4,6,7,9,10-hexahydroacridine-1,8(2H,5H)-dione (exemple 24) ;

ainsi que :

- 9-(3,4-dihydroxyphényl)-3,3,6,6-tétraméthyl-3,4,6,7,9,10-hexahydroacridine-1,8(2H,5H)-dione (exemple 2) ;
- 9-(3,4-dihydroxyphényl)-3,3,6,6,10-pentaméthyl-3,4,6,7,9,10-hexahydroacridine-1,8(2H,5H)-dione (exemple 4) ;
- 9-(4-hydroxy-3-méthoxyphényl)-3,3,6,6-tétraméthyl-3,4,6,7,9,10-hexahydroacridine-1,8(2H,5H)-dione (exemple 10) ;
- 9-(4-hydroxy-3-méthoxyphényl)-3,3,6,6,10-pentaméthyl-3,4,6,7,9,10-hexahydroacridine-1,8(2H,5H)-dione (exemple 11) ;
- 10-éthyl-9-(4-hydroxy-3-méthoxyphényl)-3,3,6,6-tétraméthyl-3,4,6,7,9,10-hexahydroacridine-1,8(2H,5H)-dione (exemple 12) ;
- 9-(4-hydroxy-3-méthoxyphényl)-3,3,6,6-tétraméthyl-10-propyl-3,4,6,7,9,10-hexahydroacridine-1,8(2H,5H)-dione (exemple 13) ;

- 9-(4-hydroxy-3-méthoxyphényl)-3,3,6,6-tétraméthyl-10-phénéthyl-3,4,6,7,9,10-hexahydroacridine-1,8(2H,5H)-dione (exemple 22).

La présente invention concerne une méthode de prévention cosmétique du vieillissement de la peau comprenant l'application sur la peau d'une composition contenant au moins un composé de formule générale (I').

La présente invention concerne un procédé de blanchiment et/ou d'éclaircissement de la peau humaine et/ou des poils et/ou des cheveux comprenant l'application sur la peau et/ou les poils et/ou les cheveux d'une composition cosmétique contenant au moins un composé de formule générale (I') essentiellement pour lequel log $P > 4$.

La présente invention concerne un procédé pour donner un aspect bronzé à la peau ou pour préparer la peau à une exposition au soleil comprenant l'application sur la peau d'une composition cosmétique contenant au moins un composé de formule générale (I') essentiellement pour lequel log $P < 3,5$.

L'invention s'étend également au procédé de synthèse des composés de formule générale (I) comme illustré par le schéma de synthèse ci-dessous :

<u>Schéma de synthèse</u>

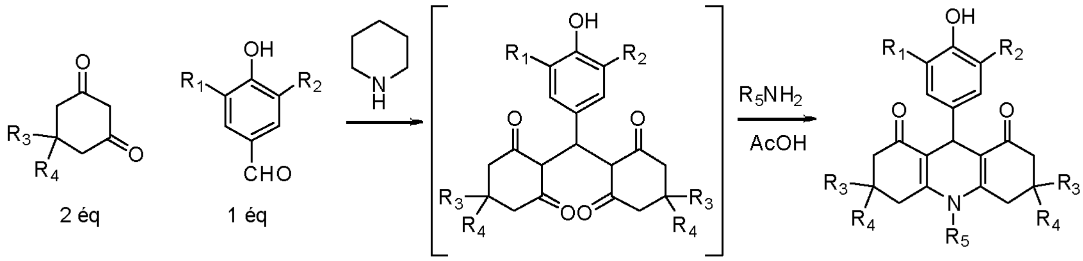


La présente invention concerne également un procédé de préparation des composés de formules générales (I) ou (I') telles que définies précédemment qui implique la réaction d'une cyclohexanedione de formule générale (II) :

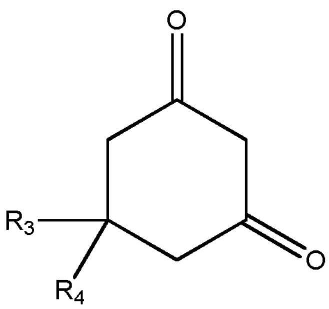

(II)

avec un 4-hydroxybenzaldéhyde de formule générale (III) :

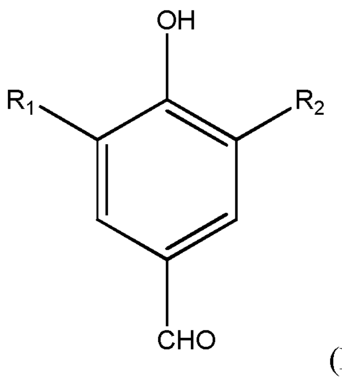

(III)

pour conduire à un produit intermédiaire de formule (IV) :

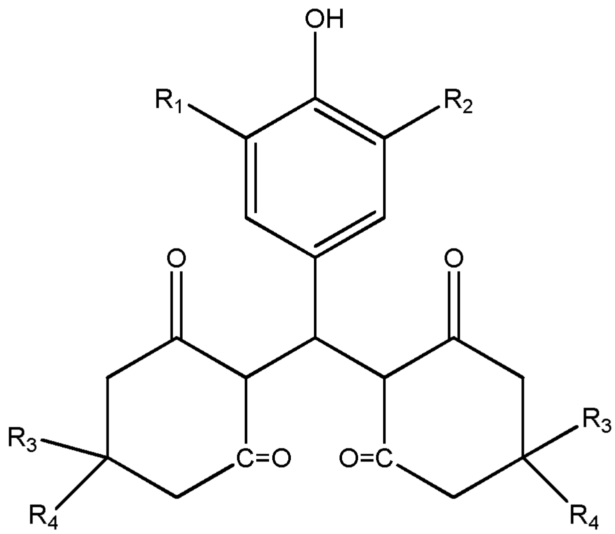

(IV)

qui est mis à réagir avec une alkylamine de formule (V) :

$R_5NH_2$ (V)

pour conduire aux composés de formule (I) correspondants, les significations des radicaux $R_1$ à $R_5$ dans les différentes formules générales (II) à (V) étant telles que définies précédemment.

Selon une autre caractéristique, la présente invention concerne un procédé dans lequel la réaction entre les composés de formules générales (II) et (III) est conduite en présence de pipéridine.

Selon encore une autre caractéristique, l'invention concerne un procédé dans lequel la réaction entre les composés de formules générales (IV) et (V) est conduite en présence d'acide acétique.

Evaluation pharmacologique

A) Test du dosage de la mélanine dans des cellules B16-F10

Principe

Il s'agit d'un test de mesure de la synthèse de mélanine par dosage colorimétrique sur une lignée cellulaire de mélanomes murins : la lignée B16-F10. Ce test permet d'évaluer le pouvoir dépigmentant ou propigmentant de principes

actifs.

Les cellules B16-F10 sont ensemencées dans des plaques 96 puits dans du milieu DMEM supplémenté de SVF (sérum de veau foetal), et incubées 24 h à 37°C, 5 % $CO_2$. Les cellules sont ensuite stimulées à l'$\alpha$-MSH 0,1 $\mu$M (pour stimuler la synthèse de mélanine, la stimulation observée est d'environ 150 %) et traitées 72 h avec les actifs à tester. Chaque concentration d'actif est testée au moins en triplicata. La mélanine totale suivie de la mélanine intracellulaire dissoute dans du tampon de lyse sont alors dosées par lecture d'absorbance à 405 nm. Les protéines totales sont dosées dans le lysat et les résultats sont exprimés en mg mélanine/mg protéines. Le pourcentage d'activité est calculé comme suit :

$$\text{\% d'activité} = \frac{\text{Moyenne normalisée du traité} - \text{Moyenne normalisée du témoin (MSH)}}{\text{Moyenne normalisée du témoin (MSH)}} \times 100$$

Une valeur négative indique une inhibition de la synthèse de la mélanine et traduit une activité dépigmentante du composé, alors qu'une valeur positive indique une induction de la synthèse de mélanine et traduit un pouvoir propigmentant de la molécule.

Matériel biologique

- lignée cellulaires B16-F10 entre P10 et P20 (mélanocytes murins) (ATCC, CRL-6475) ;
- réactifs :

  DMEM sans rouge de phénol (GIBcoBRL, 118800-028), glutamax-I supplément à 200 mM (GIBcoBRL, 35050-038), glucose (SIGMA, G7021), PBS (GIBcoBRL, 14190-094), sérum de veau foetal (Invitrogen, 10270-098), trypsine-EDTA (GIBcoBRL, 25300-054), NaOH (Sigma, S8045-500G), DMSO (Sigma, 471267-1L), Nie, Phe-Melanocyte Stimulating Hormon (Sigma, M-8764), mélanine (Sigma, M-0418), BCA-COPPER (SIGMA, B9643 et C2284), BSA (SIGMA, P0914)

B) Test pour l'étude de la capacité anti-oxydante par chimioluminescence (Photochem Analytic Jena)

Principe

Ce test est utilisé pour déterminer la capacité anti-oxydante des molécules. C'est une méthode qui génère des radicaux libres par un signal photochimique. L'intensité de l'oxydation est 1000 fois supérieure à celles obtenues en conditions normales.

La détection se fait par chimioluminescence. Elle permet l'évaluation de molécules ou extraits antioxydants hydrosolubles et liposolubles.

Les résultats sont exprimés respectivement en quantité équivalente de vitamine C ou de Trolox (acide 6-hydroxy-2,5,7,8-tétraméthylchroman-2-carboxylique ). La sensibilité est de l'ordre de la nanomole.

L'activité anti-oxydante étudiée dans ce test représente la capacité à piéger spécifiquement les anions superoxydes par chimioluminescence.

Les résultats quantifiés sont exprimés en équivalent Trolox (standard) soit en "$\mu$g de produit pour 1 $\mu$g de Trolox". Cela signifie qu'il faut une quantité x d'échantillon pour obtenir une activité équivalente à l'activité détectée pour 1 $\mu$g de standard. C'est un pouvoir antioxydant relatif à une référence ; cela nous affranchit de la concentration testée.

Génération de radicaux libres oxygénés

Le radical superoxyde : $O_2^{\circ -}$ est généré par une réaction photochimique :

$$L + h\nu\ (UV) + O_2 \rightarrow L^*\ O_2 \rightarrow L^{\circ +} + O_2^{\circ -}$$

*L* : luminol à l'état excité*

*$L^{°+}$ : radical luminol*

Détection du signal

Une partie des anions superoxydes est inhibée par les antioxydants. Les radicaux libres restants sont quantifiés par chimioluminescence.

$$L^{°+} + O_2^{°-} \rightarrow N2 + AP^{*2-} \rightarrow AP^{2-} + hv \text{ (luminescence)}$$

$AP^{*2-}$ *: aminophtalate à l'état excité*

| Nom | Conditions | Photosensibilisant | Antioxydant |
|---|---|---|---|
| Blanc | 100% $O_2^{°-}$ générés | + | - |
| Standards | gamme standard: de 1 à 3 nmol | + | vitamine C ou Trolox |
| Essai | +/- $O_2^{°-}$ générés | + | molécule x à tester |

A) Test du dosage de la mélanine dans des cellules B16-F10

Les résultats ont été regroupés dans le tableau 2 récapitulatif ci-après. Interprétation des résultats :

Une valeur négative indique une inhibition de la synthèse de mélanine, une valeur positive une stimulation de la synthèse de mélanine.

On constate que :

- les composés testés ayant un log P > 4 présentent une bonne capacité d'inhibition de la synthèse de mélanine ;
- les composés testés ayant un log P < 3,5 présentent une bonne capacité d'induction de la synthèse de mélanine.

B) Test pour l'étude de la capacité anti-oxydante par chimioluminescence (Photochem Analytic Jena)

Les résultats ont également été regroupés dans le tableau 1 récapitulatif ci-après.

L'échelle pour interpréter les résultats est la suivante :

| Produits | μg éch. pour 1 μg de Trolox | Activité |
|---|---|---|
| Vitamine C | 0,1 à 3,0 | très bonne |
| BHT | 3,01 à 50 | bonne |
| Cystéine | 50,1 à 1000 | moyenne |
| Albumine | > 1000 | faible |
| Acide lipoïque | NEGATIF | nulle |

| Exemple | Structure du composé | µg éch pour 1 µg de Trolox |
|---|---|---|
| Exemple 1 | 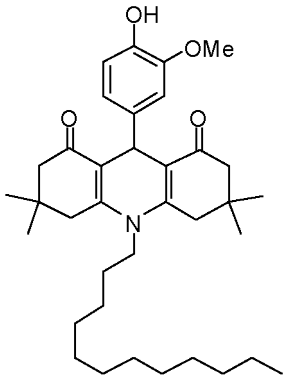 | 2,45 |
| Exemple 2 | 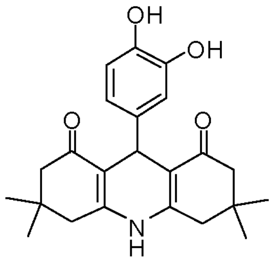 | 0,04 |
| Exemple 3 | 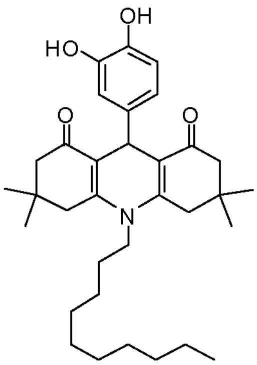 | 0,015 |
| Exemple 4 | 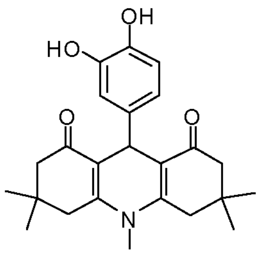 | 0,017 |
| Exemple 5 | 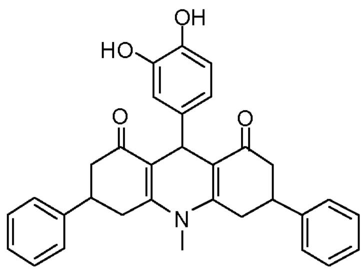 | 0,05 |

(suite)

| Exemple | Structure du composé | μg éch pour 1 μg de Trolox |
|---|---|---|
| Exemple 6 | 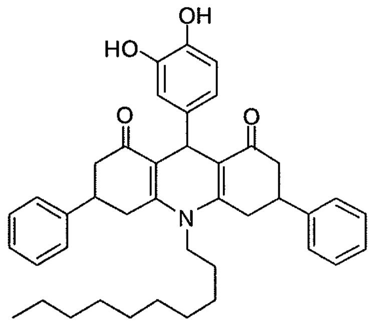 | 0,04 |
| Exemple 7 | 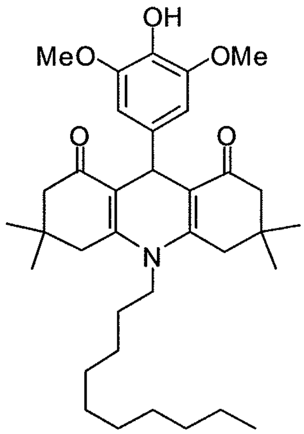 | 0,81 |
| Exemple 8 | 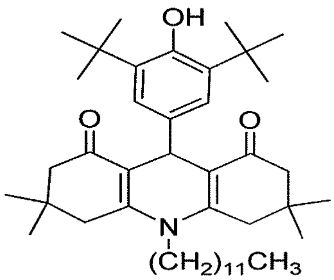 | 61,9 |
| Exemple 9 | 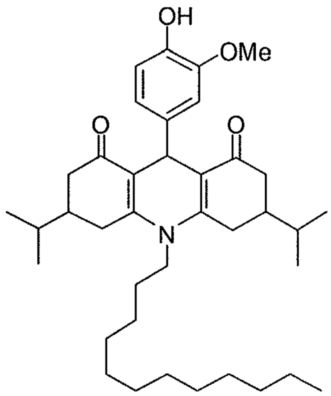 | 1,57 |

La majorité des composés a une bonne activité anti-oxydante.

La plupart des composés présentent des résultats comparables à la vitamine C. Tous les composés montrent des résultats inférieurs à 1000 μg de Trolox (62 μg étant le plus faible résultat obtenu avec l'exemple 8) ; ils ont donc tous une activité anti-oxydante intéressante.

Tableau 2

| Exemple | Structure du composé | Synthèse de mélanine sur B16 | Log P |
|---|---|---|---|
| Exemple 3 | 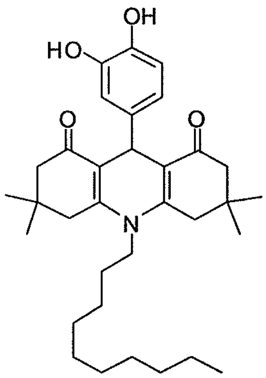 | - 46 % à 20 μM | 5,21 |
| Exemple 7 | 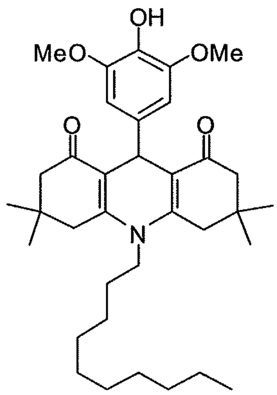 | - 56 % à 10 μM | 5,34 |
| Exemple 8 | 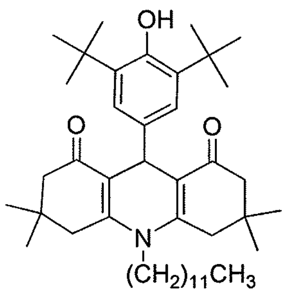 | - 46 % à 20 μM | >5 |
| Exemple 9 | 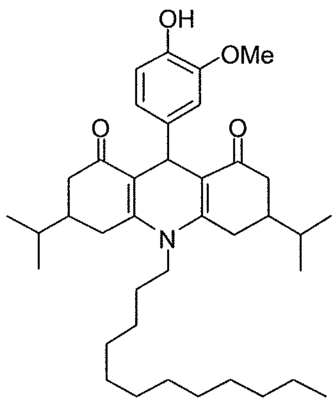 | - 49 % à 10 μM | >5 |

(suite)

| Exemple | Structure du composé | Synthèse de mélanine sur B16 | Log P |
|---|---|---|---|
| - | 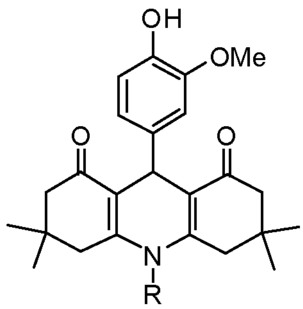 | - | - |
| Exemple 10 | H | + 29 % à 100 µM | 1,49 |
| Exemple 11 | $-CH_3$ | + 47 % à 100 µM | 1,72 |
| Exemple 12 | $-CH_2CH_3$ | + 72 % à 50 µM | 2,06 |
| Exemple 13 | $-(CH_2)_2CH_3$ | + 43 % à 50 µM | 2,55 |
| Exemple 14 | $-(CH_2)_3CH_3$ | + 45 % à 20 µM | 2,97 |
| Exemple 15 | $-(CH_2)_4CH_3$ | + 42 % à 50 µM | 3,38 |
| Exemple 16 | $-(CH_2)_5CH_3$ | -- | 3,8 |
| Exemple 17 | $-(CH_2)_6CH_3$ | - 68 % à 20 µM | 4,22 |
| Exemple 18 | $-(CH_2)_7CH_3$ | - 59 % à 5 µM | 4,63 |
| Exemple 19 | $-(CH_2)_8CH_3$ | - 42 % à 5 µM | 5,05 |
| Exemple 20 | $-(CH_2)_{10}CH_3$ | - 57 % à 5 µM | 5,89 |
| Exemple 1 | $-(CH_2)_{11}CH_3$ | - 40 % à 2 µM | 6,3 |
| Exemple 21 | $-CH_2Ph$ | + 48 % à 100 µM | 3,46 |
| Exemple 22 | $-(CH_2)_2Ph$ | -- | 3,74 |
| Exemple 23 | $-(CH_2)_3Ph$ | - 37 % à 10 µM | 4,15 |
| Exemple 24 | $-CH_2CH(Ph)_2$ | - 37 % à 5 µM | 5,32 |

Rappel : Une valeur négative indique une inhibition de la synthèse de mélanine, une valeur positive une stimulation de la synthèse de mélanine.

EXEMPLES DE SYNTHESE

Exemple 1

- 10-dodécyl-9-(4-hydroxy-3-méthoxyphényl)-3,3,6,6-tétraméthyl-3,4,6,7,9,10-hexahydroacridine-1,8(2H,5H)-dione

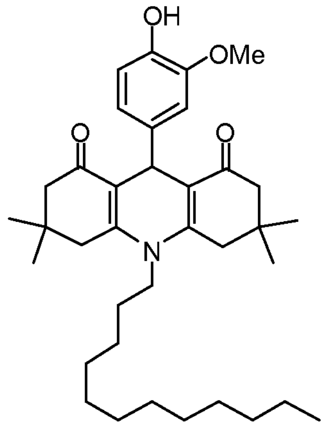

Procédure générale

A une suspension de 2,80g de 5,5-diméthyl-1,3-cyclohexanedione (dimédone, 20 mmol) et de 1,52 g de vanilline (10 mmol) dans 40 mL d'éthanol, 49,45 µL de pipéridine (0,5 mmol) sont ajoutés. Le mélange est porté à reflux à une température de 110°C et on observe la solubilisation des particules en suspension en une solution limpide orange. Après 4 h de réaction, la solution est refroidie à température ambiante pendant 10 min, puis est placée 10 min dans un bain de glace, et le précipité blanc obtenu est filtré avec un minimum d'éthanol froid et du n-pentane. Le solide est séché à l'étuve à vide pendant au moins une nuit sous 50 mbar et à 40°C afin d'obtenir un solide blanc avec un rendement moyen de 86 %.

Au mélange de 1,24 g de produit intermédiaire (3 mmol) et de 0,55 g de n-dodécylamine (3 mmol), 15 mL d'acide acétique sont ajoutés. Le mélange est porté à reflux à une température de 140°C et on observe la solubilisation des particules en suspension en une solution limpide jaune. Après 4 h de réaction, la solution est refroidie à température ambiante pendant 10 min et est évaporée à sec. Une chromatographie est réalisée en utilisant un gradient de 90:10 à 50:50 en heptane/acétate d'éthyle. La fraction pure est récupérée et évaporée. Le solide est séché à l'étuve à vide au moins une nuit sous 50 mbar et à 40°C afin d'obtenir un solide jaune avec un rendement moyen de 50 %.

$^{1}$H-RMN (400 MHz, $CDCl_3$, δ) : 0,88 (t, 3H), 1,00 (s, 6H), 1,09 (s, 6H), 1,27 (s, 16H), 1,33 (m, 2H), 1,59 (m, 2H), 2,22 (s, 4H), 2,38 (d, syst. AB, 2H), 2,51 (d, syst. AB, 2H), 3,62 (t, 2H), 3,85 (s, 3H), 5,17 (s, 1 H), 5,43 (s, 1 H), 6,50 (dd, 1 H), 6,67 (d, 1H),7,01 (s, 1 H).

$^{13}$C-RMN (100 MHz, $CDCl_3$, δ) : 14,0, 22,6, 26,5, 27,5, 29,2, 29,4, 29,6, 31,5, 31,7, 32,3, 40,4, 44,7, 49,8, 56,1, 104,5, 115,2, 132,8, 137,2, 146,4, 149,6, 195,6.

MS (ESI$^{+}$) : 564,4 $[M+H]^{+}$.

Rf (cyclohexane/AcOEt ; 1/1) : 0,31.

Exemple 2

- 9-(3,4-dihydroxyphényl)-3,3,6,6-tétraméthyl-3,4,6,7,9,10-hexahydroacridine-1,8(2H,5H)-dione

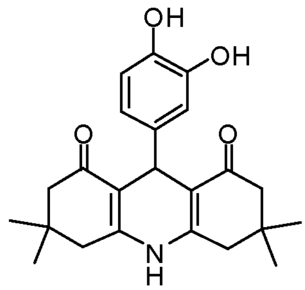

$^{1}$H-RMN (400 MHz, DMSO-d6, δ) : 0,88 (s, 6H), 1,00 (s, 6H), 2,00-2,40 (m, 8H), 4,64 (s, 1 H), 6,38 (s, 1 H), 6,47 (s, 1 H), 6,59 (s, 1 H), 8,51 (s, 2H, OH), 9,17 (s, 1 H, NH).

$^{13}$C-RMN (100 MHz, DMSO d6, δ) : 26,4, 29,0, 31,6, 32,0, 40,0, 50,3, 111,8, 114,6, 115,4, 118,6, 138,4, 142,8, 144,1,

148,6, 194,2.
MS ($APCI^+$): 282,2 $[M+H]^+$

Exemple 3

- 10-décyl-9-(3,4-dihydroxyphényl)-3,3,6,6-tétraméthyl-3,4,6,7,9,10-hexahydroacridine-1,8(2H,5H)-dione

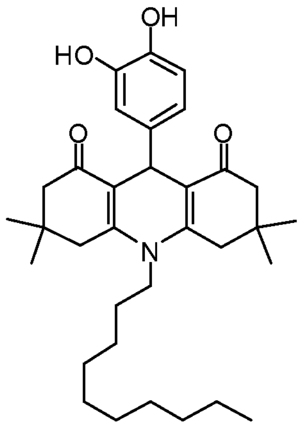

$^1$H-RMN (400 MHz, DMSO-d6, δ) : 0,85 (t, 3H), 0,92 (s, 6H), 1,01 (s, 6H), 1,23 (s, XH), 1,49 (m, 2H), 2,08 (dd, 4H), 2,60 (dd, 4H), 3,67 (t, 2H), 4,64 (s, 1 H), 6,26 (s, 1 H), 6,44 (s, 1 H), 6,58 (s, 1 H), 8,42 (s, 1 H, OH), 8,51 (s, 1 H, OH).
$^{13}$C-RMN (100 MHz, DMSO-d6, δ) : 13,8, 21,9, 25,6, 26,9, 28,6, 29,5, 30,5, 31,1, 31,9, 40,0, 44,1, 49,5, 113,7, 114,6, 115,1, 117,1, 137,2, 142,8, 144,2, 150,9, 194,9.
MS ($ESI^+$) : 522,3 $[M+H]^+$.
Rf (cyclohexane/EtOAc ; 1/1) : 0,16.

Exemple 4

- 9-(3,4-dihydroxyphényl)-3,3,6,6,10-pentaméthyl-3,4,6,7,9,10-hexahydroacridine-1,8(2H,5H)-dione

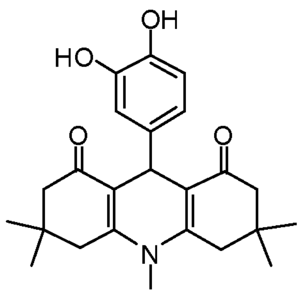

$^1$H-RMN (400 MHz, DMSO-d6, δ) : 0,96 (s, 6H), 0,99 (s, 6H), 2,11 (dd, 4H), 2,40 (d, syst. AB, 2H), 2,74 (d, syst. AB, 2H), 3,24 (s, 3H), 4,86 (s, 1 H), 6,28 (d, 1 H, 6,46 (d, 1 H), 6,54 (s, 1 H), 8,42 (s, 1 H, OH), 8,52 (s, 1 H, OH). $^{13}$C-RMN (100 MHz, DMSO-d6, δ) : 26,2, 27,7, 27,9, 28,2, 29,5, 32,0, 33,1, 40,0, 49,5, 113,1, 114,8, 114,9, 117,3, 137,1, 142,9, 144,2, 152,0, 194,6. MS ($ESI^+$): 396,2 $[M+H]^+$.
Rf ($CHCl_3$/MeOH ; 95/5) : 0,26.

Exemple 5

- 9-(3,4-dihydroxyphényl)-10-méthyl-3,6-diphényl-3,4,6,7,9,10-hexahydroacridine-1,8(2H,5H)-dione

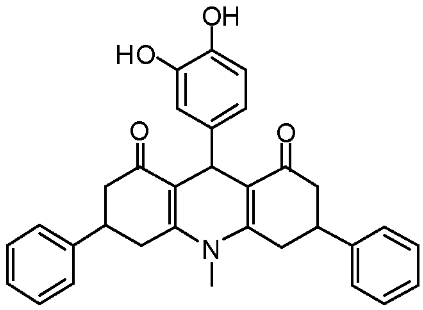

MS ($ESI^+$) : 492,2 $[M+H]^+$.
Rf (EtOAc) : 0,76.

Exemple 6

- 10-décyl-9-(3,4-dihydroxyphéyl)-3,6-diphéyl-3,4,6,7,9,10-hexahydroacridine-1,8(2H,5H)-dione

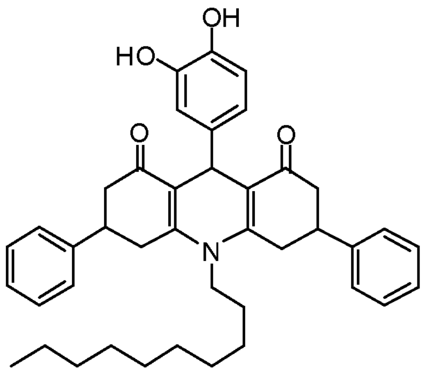

$^1$H-RMN (400 MHz, $CDCl_3$, $\delta$) : 0,87 (t, 3H), 1,21 (s, 14H), 1,58 (m, 2H), 1,70 (m, 2H), 2,54-3,02 (m, 8H), 3,24-3,63 (m, 4H), 5,26 (s, 1 H), 6,56 (m, 2H), 6,95 (d, 1 H), 7,28 (m, 10H).
MS ($ESI^+$) : 618,3 $[M+H]^+$.

Exemple 7

- 10-décyl-9-(4-hydroxy-3,5-diméthoxyphényl)-3,3,6,6-tétraméthyl-3,4,6,7,9,10-hexahydroacridine-1,8(2H,5H)-dione

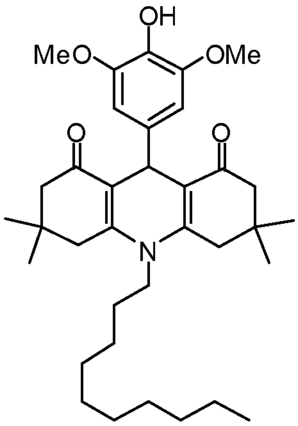

$^1$H-RMN (400 MHz, $CDCl_3$, δ) : 0,88 (t, 3H), 1,02 (s, 6H), 1,10 (s, 6H), 1,27 (s, 12H), 1,31 (m, 2H), 1,59 (m, 2H), 2,23 (s, 4H), 2,40 (d, syst. AB, 2H), 2,50 (d, syst. AB, 2H), 3,62 (t, 2H), 3,80 (s, 6H), 5,20 (s, 1 H), 6,52 (s, 2H).
$^{13}$C-RMN (100 MHz, $CDCl_3$, δ) : 14,0, 22,6, 26,5, 27,5, 29,2, 29,4, 29,6, 31,5, 31,7, 32,3, 40,4, 44,7, 49,8, 56,1, 104,5, 115,2, 132,8, 137,2, 146,4, 149,6, 195,6.
MS (ESI$^+$) : 566,4 $[M+H]^+$.
Rf (cyclohexane/AcOEt ; 1/1) : 0,18.

Exemple 8

- 10-dodécyl-9-(3,5-di-tert-butyl-4-hydroxyphényl)-3,3,6,6-tétraméthyl-3,4,6,7,9,10-hexahydroacridine-1,8(2H,5H)-dione

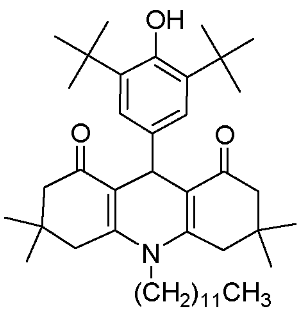

$^1$H-RMN (400 MHz, $CDCl_3$, δ) : 0,88 (t, 3H), 1,02 (s, 6H), 1,09 (s, 6H), 1,26 (s, 18H), 1, 35 (s, 18H), 1,56 (m, 2H), 2,22 (m, 4H), 2,46 (m, 4H), 3,56 (t, 2H), 4,91 (s, 1 H), 5,20 (s, 1 H), 7,00 (s, 2H).
$^{13}$C-RMN (100 MHz, $CDCl_3$, δ) : 26,53, 26,87, 27,59, 29,15, 29,29, 29,41, 29,50, 29,57, 30,43, 30,93, 31,86, 32,44, 34,17, 40,48, 44,89, 50,00, 115,57, 124,06, 134,36, 136,36, 149,69, 151,69, 195,65.
MS (ESI$^+$) : 646.5 $[M+H]^+$.
Rf (cyclohexane/EtOAc ; 7/3) : 0,13.

Exemple 9

- 10-dodécyl-9-(4-hydroxy-3-méthoxyphényl)-3,6-diisopropyl-3,4,6,7,9,10-hexahydroacridine-1,8(2H,5H)-dione

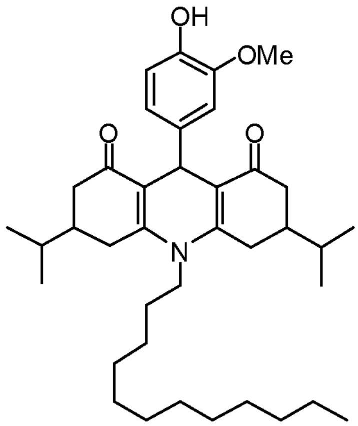

$^1$H-RMN (400 MHz, $CDCl_3$, δ) : 0,86-0,96 (m, 15H), 1,26 (m, 19H), 1,60-2,47 (m, 13H), 2,52 (m, 1 H), 2,56 (m, 1 H), 3,66 (m, 2H), 3,87 (s, 3H), 5,18 (s, 1 H), 5,47 (s, 1 H), 6,44 (dd, 1 H), 6,67 (d, 1 H), 7,07 (d, 1 H).
MS (ESI$^+$) : 592.4 [M+H]$^+$.
Rf (cyclohexane/EtOAc ; 1/1) : 0,46.

Exemple 10

- 9-(4-hydroxy-3-méthoxyphényl)-3,3,6,6-tétraméthyl-3,4,6,7,9,10-hexahydroacridine-1,8(2H,5H)-dione

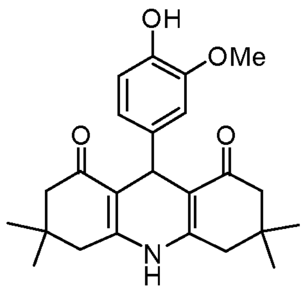

$^1$H-RMN (300 MHz, DMSO-d6, δ) : 0,80 (s, 6H), 1,01 (s, 6H), 2,00-2,40 (m, 8H), 3,65 (s, 3H), 4,72 (s, 1 H), 6,50 (2d, 2H), 6,70 (s, 1 H), 8,58 (s, 1 H, OH), 9,23 (s, 1 H, NH).
$^{13}$C-RMN (75 MHz, DMSO-d6, δ) : 26,74, 29,53, 32,24, 32,74, 50,68, 55,85, 112,11, 112,58, 115,11, 120,13, 138,86, 144,69, 147,05, 149,32, 194,80. MS (ES$^+$) : 418,2 [M+Na]$^+$ ; 813,5 [2M+Na]$^+$.
MS (ES$^-$) : 394,2 [M-H]$^-$.
Pf = 309,8°C.

Exemple 11

- 9-(4-hydroxy-3-méthoxyphényl)-3,3,6,6,10-pentaméthyl-3,4,6,7,9,10-hexahydroacridine-1,8(2H,5H)-dione

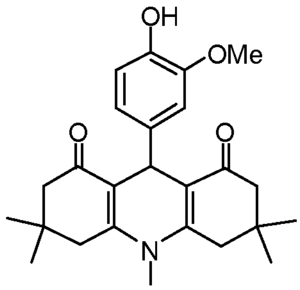

$^{1}$H-RMN (400 MHz, $CDCl_3$, δ) : 1,03 (s, 6H), 1,07 (s, 6H), 2,22 (s, 4H), 2,40 (m, 4H), 3,25 (s, 3H), 3,84 (s, 3H), 5,17 (s, 1 H), 5,29 (s, 1 H), 6,45 (dd, 1 H), 6,67 (d, 1 H), 7,02 (d, 1 H).
$^{13}$C-RMN (100 MHz, $CDCl_3$, δ) : 28,59, 28,64, 31,16, 32,63, 33,41, 40,50, 49,83, 55,79, 111,59, 113,70, 115,10, 118,72, 137,95, 143,62, 145,90, 151,00, 195,55. MS (ESI$^+$) : 410,2 [M+H]$^+$.
Rf (DCM/MeOH ; 95/5) : 0,33.

Exemple 12

- 10-éthyl-9-(4-hydroxy-3-méthoxyphényl)-3,3,6,6-tétraméthyl-3,4,6,7,9,10-hexahydroacridine-1,8(2H,5H)-dione

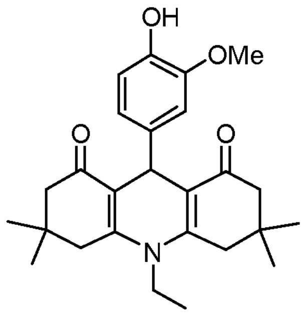

$^{1}$H-RMN (300 MHz, DMSO-d6, δ) : 0,91 (s, 6H), 1,03 (s, 6H), 1,18 (t, 3H), 2,07 (d, 2H), 2,17 (d, 2H), 2,46 (d, 2H), 2,69 (d, 2H), 3,63 (s, 3H), 3,78 (q, 2H), 4,91 (s, 1 H), 6,50 (2d, 2H), 6,60 (s, 1 H), 8,59 (s, 1 H, OH).
$^{13}$C-RMN (75 MHz, DMSO-d6, δ) : 16,55, 27,05, 29,37, 30,51, 32,38, 49,91, 55,69, 111,60, 114,06, 115,09, 119,84, 137,87, 144,69, 147,24, 151,22, 195,39. MS (ES$^+$) : 446,1 [M+Na]$^+$ ; 869,4 [2M+Na]$^+$.
MS (ES$^-$) : 422,3 [M-H]$^-$.

Exemple 13

- 9-(4-hydroxy-3-méthoxyphényl)-3,3,6,6-tétraméthyl-10-propyl-3,4,6,7,9,10-hexahydroacridine-1,8(2H,5H)-dione

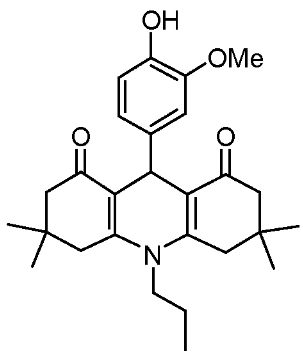

$^{1}$H-RMN (300 MHz, DMSO-d6, δ) : 0,85 (t, 3H), 0,92 (s, 6H), 1,03 (s, 6H), 1,52 (m, 2H), 2,08 (d, 2H, syst. AB), 2,19 (d, 2H, syst. AB), 2,44 (d, 2H, syst. AB), 2,67 (d, 2H, syst. AB), 3,63 (s, 3H), 3,68 (t, 2H), 4,95 (s, 1 H), 6,51 (2d, 2H), 6,60 (s, 1 H), 8,59 (s, 1 H, OH).
$^{13}$C-RMN (75 MHz, DMSO-d6, δ) : 11,00, 24,52, 27,09, 29,41, 30,19, 32,21, 32,38, 46,01, 49,93, 55,67, 111,58, 113,89, 115,11, 119,72, 137,59, 144,68, 147,23, 151,52, 195,51.
MS (ES$^+$) : 460,1 [M+Na]$^+$ ; 897,7 [2M+Na]$^+$.
Pf : 259,5°C.

Exemple 14

- 10-butyl-9-(4-hydroxy-3-méthoxyphényl)-3,3,6,6-tétraméthyl-3,4,6,7,9,10-hexahydroacridine-1,8(2H,5H)-dione

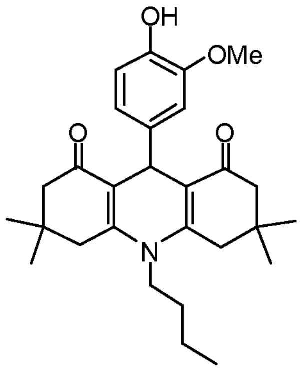

$^{1}$H-RMN (400 MHz, $CDCl_3$, δ) : 1,00 (m, 12H), 1,09 (s, 3H), 1,40 (m, 2H), 1,60 (m, 3H), 2,22 (s, 4H), 2,47 (m, 4H), 3,63 (t, 2H), 3,85 (s, 3H), 5,17 (s, 1H), 5,45 (s, 1 H), 6,50 (2d, 1 H), 6,68 (d, 1H), 7,02 (d, 1 H).
$^{13}$C-RMN (100 MHz, $CDCl_3$, δ) : 13,79, 19,86, 27,86, 29,33, 31,35, 32,50, 33,55, 40,43, 44,56, 49,91, 55,79, 111,55, 113,55, 115,57, 119,03, 138,20, 143,55, 145,87, 149,89, 195,73.
MS (ESI$^+$) : 452,3 [M+H]$^+$.
Rf (DCM/MeOH ; 98/2) : 0,50.

Exemple 15

- 9-(4-hydroxy-3-méthoxyphényl)-3,3,6,6-tétraméthyl-10-pentyl-3,4,6,7,9,10-hexahydroacridine-1,8(2H,5H)-dione

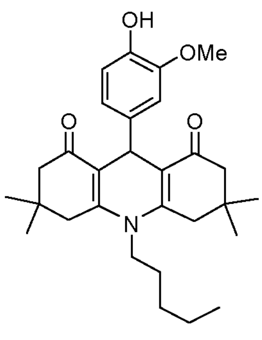

$^{1}$H-RMN (300 MHz, $CDCl_3$, δ) : 0,90 (t, 3H), 1,00 (s, 6H), 1,09 (s, 6H), 1,30 (m, 4H), 1,62 (m, 2H), 2,24 (s, 4H), 2,39 (d, syst. AB, 2H), 2,53 (d, syst. AB, 2H), 3,64 (t, 2H), 3,87 (s, 3H), 5,19 (s, 1 H), 5,47 (s l, 1H), 6,52 (dd, 1 H), 6,68 (d, 1 H), 7,03 (d, 1 H).
$^{13}$C-RMN (75 MHz, $CDCl_3$, δ) : 14,3, 22,80, 28,24, 29,14, 29,78, 31,61, 31,75, 32,67, 32,91, 40,85, 45,18, 50,35, 56,21, 111,97, 113,96, 115,98, 119,44, 138,63, 143,98, 146,30, 150,31, 196,12.
MS (ES$^+$) : 466,2 [M+H]$^+$ ; 953,6 [2M+Na]$^+$.

Pf : 194,5°C.

Exemple 16

- 10-hexyl-9-(4-hydroxy-3-méthoxyphényl)-3,3,6,6-tétraméthyl-3,4,6,7,9,10-hexahydroacridine-1,8(2H,5H)-dione

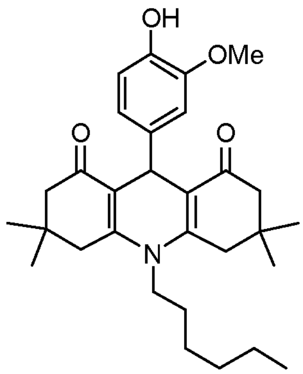

$^{1}$H-RMN (300 MHz, $CDCl_3$, δ) : 0,90 (t, 3H), 1,02 (s, 6H), 1,09 (s, 6H), 1,30 (m, 6H), 1,62 (m, 2H), 2,24 (s, 4H), 2,38 (d, syst. AB, 2H), 2,53 (d, syst. AB, 2H), 3,64 (t, 2H), 3,87 (s, 3H), 5,18 (s, 1 H), 5,47 (s l, 1H), 6,52 (dd, 1 H), 6,69 (d, 1 H), 7,03 (d, 1 H).
$^{13}$C-RMN (75 MHz, DMSO-d6, δ) : 16,55, 27,05, 29,37, 30,51, 32,38, 40,91, 55,69, 111,60, 114,06, 115,09, 119,84, 137,87, 144,68, 147,24, 151,22, 195,39. MS (ES$^+$) : 480,2 $[M+Na]^+$ ; 981,7 $[2M+Na]^+$.
Pf : 177,7°C.

Exemple 17

- 10-heptyl-9-(4-hydroxy-3-méthoxyphényl)-3,3,6,6-tétraméthyl-3,4,6,7,9,10-hexahydroacridine-1,8(2H,5H)-dione

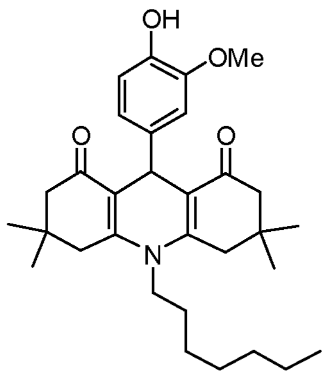

$^{1}$H-RMN (300 MHz, $CDCl_3$, δ) : 0,92 (t, 3H), 1,02 (s, 6H), 1,10 (s, 6H), 1,30 (m, 8H), 1,61 (m, 2H), 2,24 (s, 4H), 2,39 (d, syst. AB, 2H), 2,53 (d, syst. AB, 2H), 3,63 (t, 2H), 3,87 (s, 3H), 5,18 (s, 1 H), 5,46 (s l, 1 H), 6,52 (dd, 1 H), 6,69 (d, 1 H), 7,03 (d, 1 H).
$^{13}$C-RMN (75 MHz, $CDCl_3$, δ) : 14,45, 22,95, 27,01, 28,23, 29,34, 29,79, 31,77, 31,89, 32,06, 40,85, 45,21, 50,35, 56,21, 111,97, 113,96, 115,98, 119,44, 138,64, 143,98, 146,29, 150,29, 196,11.
MS (ES$^+$) : 494,2 $[M+H]^+$ ; 1009,7 $[2M+Na]^+$.
Pf : 154,8°C.

Exemple 18

- 9-(4-hydroxy-3-méthoxyphényl)-3,3,6,6-tétraméthyl-10-octyl-3,4,6,7,9,10-hexahydroacridine-1,8(2H,5H)-dione

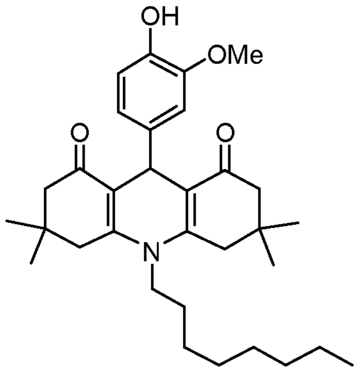

$^{1}$H-RMN (300 MHz, $CDCl_3$, δ) : 0,91 (t, 3H), 1,02 (s, 6H), 1,11 (s, 6H), 1,30 (m, 10H), 1,61 (m, 2H), 2,24 (s, 4H), 2,39 (d, syst. AB, 2H), 2,53 (d, syst. AB, 2H), 3,63 (t, 2H), 3,87 (s, 3H), 5,18 (s, 1 H), 5,46 (s l, 1H), 6,52 (dd, 1 H), 6,69 (d, 1 H), 7,03 (d, 1 H).
RMN $^{13}$C (75 MHz, $CDCl_3$) : δ : 14,47; 23,02; 27,04; 28,23; 29,53; 29,63; 29,79; 31,77; 31,87; 32,11; 32,90; 40,85; 45,21; 50,35; 56,21; 111,97; 113,97; 115,98; 119,44; 138,63; 143,98; 146,29; 150,28; 196,12.
MS ($ES^+$): 508,2 $[M+H]^+$ ; 1037,8 $[2M+Na]^+$.
Pf : 154,2°C.

Exemple 19

- 9-(4-hydroxy-3-méthoxyphényl)-3,3,6,6-tétraméthyl-10-nonyl-3,4,6,7,9,10-hexahydroacridine-1,8(2H,5H)-dione

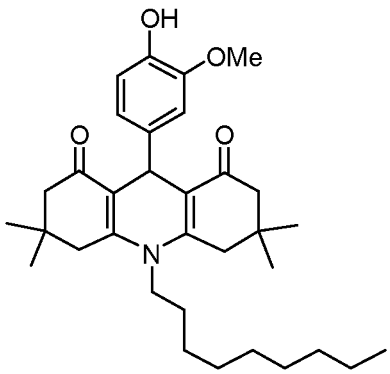

$^{1}$H-RMN (300 MHz, $CDCl_3$, δ) : 0,91 (t, 3H), 1,02 (s, 6H), 1,11 (s, 6H), 1,30 (m, 12H), 1,63 (m, 2H), 2,24 (s, 4H), 2,39 (d, syst. AB, 2H), 2,53 (d, syst. AB, 2H), 3,63 (t, 2H), 3,87 (s, 3H), 5,19 (s, 1 H), 5,43 (s l, 1H), 6,52 (dd, 1 H), 6,69 (d, 1 H), 7,03 (d, 1 H).
$^{13}$C-RMN (75 MHz, $CDCl_3$, δ) : 14,47, 23,03, 27,03, 28,23, 29,58, 29,67, 29,79, 29,83, 31,77, 31,88, 32,22, 32,67, 40,85, 45,21, 50,35, 56,20, 111,96, 113,98, 115,97, 119,45, 138,63, 143,99, 146,30, 150,28, 196,11.
MS ($ES^+$) : 522,2 $[M+H]^+$ ; 1065,8 $[2M+Na]^+$.
Pf : 127,0°C.

Exemple 20

- 9-(4-hydroxy-3-méthoxyphényl)-3,3,6,6-tétraméthyl-10-undécyl-3,4,6,7,9,10-hexahydroacridine-1,8(2H,5H)-dione

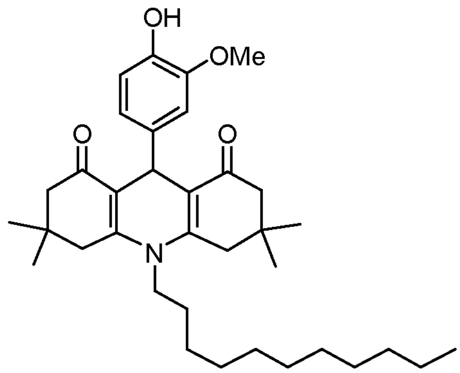

$^1$H-RMN (300 MHz, $CDCl_3$, δ) : 0,90 (t, 3H), 1,02 (s, 6H), 1,10 (s, 6H), 1,32 (m, 16H), 1,61 (m, 2H), 2,24 (s, 4H), 2,39 (d, syst. AB, 2H), 2,53 (d, syst. AB, 2H), 3,63 (t, 2H), 3,87 (s, 3H), 5,18 (s, 1 H), 5,45 (s l, 1H), 6,52 (dd, 1 H), 6,69 (d, 1 H), 7,03 (d, 1 H).
$^{13}$C-RMN (75 MHz, $CDCl_3$, δ) : 14,50, 23,06, 27,04, 28,23, 29,67, 29,70, 29,79, 29,89, 29,93, 29,98, 31,77, 31,87, 32,27, 32,90, 40,85, 45,21, 50,34, 56,21, 111,96, 113,97, 115,98, 119,44, 138,63, 143,98, 146,29, 150,28, 196,11.
MS ($ES^+$) : 550,3 $[M+H]^+$ ; 572,2 $[M+Na]^+$.
Pf : 118,9°C.

Exemple 21

- 10-benzyl-9-(4-hydroxy-3-méthoxyphényl)-3,3,6,6-tétraméthyl-3,4,6,7,9,10-hexahydroacridine-1,8(2H,5H)-dione

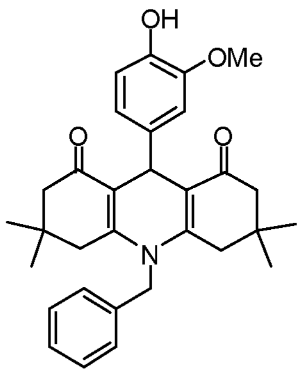

$^1$H-RMN (400 MHz, $CDCl_3$, δ) : 0,89 (s, 6H), 0,99 (s, 6H), 2,20 (s, 4H), 2,39 (m, 4H), 3,85 (s, 3H), 4,89 (s, 2H), 5,24 (s, 1 H), 6,56 (d, 1 H), 6,71 (d, 1 H), 7,07 (s, 1 H), 7,16 (d, 2H), 7,39 (m, 3H).
$^{13}$C-RMN (100 MHz, $CDCl_3$, δ) : 28,04, 28,59, 31,63, 32,66, 40,22, 48,70, 49,95, 50,74, 55,82, 111,85, 113,61, 115,40, 119,39, 125,33, 127,87, 127,96, 129,20, 137,01, 138,26, 143,66, 145,88, 150,34, 195,84.
MS ($ESI^+$) : 486,2 $[M+H]^+$.
Rf (cyclohexane/EtOAc ; 1/1) : 0,46.

Exemple 22

- 9-(4-hydroxy-3-méthoxyphényl)-3,3,6,6-tétraméthyl-10-phénéthyl-3,4,6,7,9,10-hexahydroacridine-1,8(2H,5H)-dione

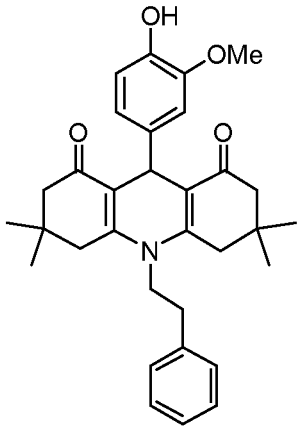

$^{1}$H-RMN (300 MHz, $CDCl_3$, δ) : 0,89 (s, 6H), 1,10 (s, 6H), 2,24 (s, 4H), 2,45 (m, 4H, syst. AB), 2,89 (dd, 2H), 3,88 (s, 3H), 3,91 (dd, 2H), 5,21 (s, 1 H), 5,49 (s, 1 H), 6,56 (dd, 1 H), 6,72 (dd, 1 H), 7,07 (d, 1 H), 7,17 (dd, 2H), 7,35 (m, 3H).
$^{13}$C-RMN (75 MHz, $CDCl_3$, δ) : 27,68, 28,08, 29,97, 31,77, 32,79, 38,22, 41,01, 46,57, 50,30, 56,25, 112,12, 113,98, 116,12, 119,48, 127,68, 129,01, 129,26, 129,43, 137,38, 138,58, 144,06, 146,34, 149,95, 196,16.
MS (ES$^+$) : 500,2 $[M+H]^+$ ; 1021,8 $[2M+Na]^+$.

Exemple 23

- 9-(4-hydroxy-3-méthoxyphényl)-3,3,6,6-tétraméthyl-10-(3-phénylpropyl)-3,4,6,7,9,10-hexahydroacridine-1,8(2H,5H)-dione

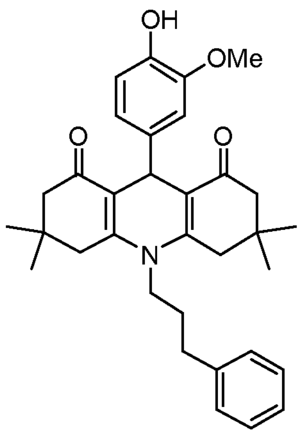

$^{1}$H-RMN (300 MHz, $CDCl_3$, δ) : 0,89 (s, 6H), 1,02 (s, 6H), 1,93 (m, 2H), 2,26 (s, 4H), 2,33 (m, 4H, syst. AB), 2,71 (dd, 2H), 3,61 (m, 2H), 3,84 (s, 3H), 5,15 (s, 1 H), 5,47 (s large, 1 H), 6,53 (dd, 1 H), 6,70 (dd, 1 H), 7,01 (d, 1 H), 7,20 (dd, 2H), 7,35 (m, 3H).
$^{13}$C-RMN (75 MHz, $CDCl_3$, δ) : 28,01, 29,79, 31,84, 32,74, 32,98, 33,06, 40,56, 44,08, 50,30, 56,19, 111,99, 114,06, 116,01, 119,45, 127,07, 128,84, 129,20, 138,72, 140,49, 143,98, 146,28, 150,08, 196,08.
MS (ES$^+$) : 514,3 $[M+H]^+$ ; 1049,9 $[2M+Na]^+$.

Exemple 24

- 10-(2,2-diphényléthyl)-9-(4-hydroxy-3-méthoxyphényl)-3,3,6,6-tétraméthyl-3,4,6,7,9,10-hexahydroacridine-1,8(2H,5H)-dione

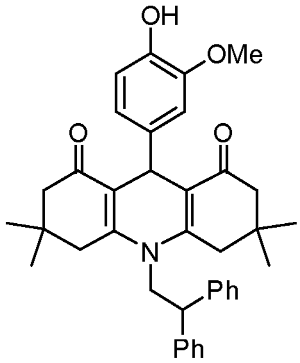

$^1$H-RMN (300 MHz, $CDCl_3$, δ) : 0,81 (s, 6H), 1,01 (s, 6H), 2,19 (s, 4H), 2,27 (m, 4H, syst. AB), 3,88 (s, 3H), 3,92 (t, 1 H), 4,36 (d, 2H), 5,24 (s, 1 H), 5,53 (s I, 1 H), 6,43 (dd, 1 H), 6,78 (dd, 1 H), 7,03 (d, 1 H), 7,17-7,35 (m, 10H).
$^{13}$C-RMN (75 MHz, $CDCl_3$, δ) : 27,29, 30,49, 31,22, 32,40, 41,17, 50,25, 50,29, 54,26, 56,25, 112,18, 113,80, 115,61, 119,44, 127,79, 128,31, 129,40, 138,26, 141,63, 144,01, 146,34, 150,59, 196,39.
MS ($ES^+$) : 576,2 $[M+H]^+$ ; 1151,7 $[2M+H]^+$.

La composition topique selon l'invention est caractérisée en ce que la quantité de composé formule (I') varie entre 0,01 % et 10 % en poids et de préférence de 0,1 % à 5% en poids par rapport au poids total de la composition.

## Revendications

1. Composé de formule générale (I) :

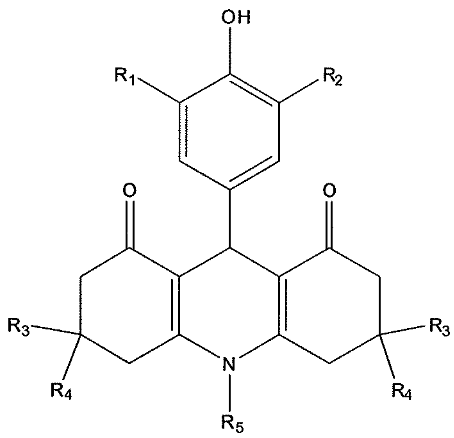

(I)

dans laquelle :

- $R_1$ et $R_2$ représentent simultanément ou indépendamment H, OH, $OCH_3$ ou un radical alkyle en $C_1$-$C_5$,
- $R_3$ et $R_4$ représentent simultanément $CH_3$,
- ou $R_3$ représente H et $R_4$ représente $CH_3$, $CH_2CH_3$ ou un radical isopropyle ou un radical phényle,
- $R_5$ représente un radical alkyle en $C_4$-$C_{24}$ ou un radical 3-phénylpropanyle ou 2,2-diphényléthanyle.

2. Composé de formule générale (I) selon la revendication 1, **caractérisé en ce que** $R_1$ représente H et $R_2$ représente $OCH_3$.

3. Composé de formule générale (I) selon l'une des revendications 1 ou 2, **caractérisé en ce que** $R_3$ et $R_4$ représentent simultanément $CH_3$.

4. Composé de formule générale (I) selon l'une des revendications 1 à 3, **caractérisé en ce qu'**il est choisi parmi l'un des composés suivants :

- 10-dodécyl-9-(4-hydroxy-3-méthoxyphényl)-3,3,6,6-tétraméthyl-3,4,6,7,9,10-hexahydroacridine-1,8(2H,5H)-dione (exemple 1) ;
- 10-décyl-9-(3,4-dihydroxyphényl)-3,3,6,6-tétraméthyl-3,4,6,7,9,10-hexahydroacridine-1,8(2H,5H)-dione (exemple 3) ;
- 9-(3,4-dihydroxyphényl)-10-méthyl-3,6-diphényl-3,4,6,7,9,10-hexahydroacridine-1,8(2H,5H)-dione (exemple 5)
- 10-décyl-9-(3,4-dihydroxyphéyl)-3,6-diphéyl-3,4,6,7,9,10-hexahydroacridine-1,8(2H,5H)-dione (exemple 6)
- 10-décyl-9-(4-hydroxy-3,5-diméthoxyphényl)-3,3,6,6-tétraméthyl-3,4,6,7,9,10-hexahydroacridine-1,8(2H,5H)-dione (exemple 7) ;
- 10-dodécyl-9-(3,5-di-tert-butyl-4-hydroxyphényl)-3,3,6,6-tétraméthyl-3,4,6,7,9,10-hexahydroacridine-1,8(2H,5H)-dione (exemple 8) ;
- 10-dodécyl-9-(4-hydroxy-3-méthoxyphényl)-3,6-düsopropyl-3,4,6,7,9,10-hexahydroacridine-1,8(2H,5H)-dione (exemple 9) ;
- 10-butyl-9-(4-hydroxy-3-méthoxyphényl)-3,3,6,6-tétraméthyl-3,4,6,7,9,10-hexahydroacridine-1,8(2H,5H)-dione (exemple 14) ;
- 9-(4-hydroxy-3-méthoxyphényl)-3,3,6,6-tétraméthyl-10-pentyl-3,4,6,7,9,10-hexahydroacridine-1,8(2H,5H)-dione (exemple 15) ;
- 10-hexyl-9-(4-hydroxy-3-méthoxyphényl)-3,3,6,6-tétraméthyl-3,4,6,7,9,10-hexahydroacridine-1,8(2H,5H)-dione (exemple 16) ;
- 10-heptyl-9-(4-hydroxy-3-méthoxyphényl)-3,3,6,6-tétraméthyl-3,4,6,7,9,10-hexahydroacridine-1,8(2H,5H)-dione (exemple 17) ;
- 9-(4-hydroxy-3-méthoxyphényl)-3,3,6,6-tétraméthyl-10-octyl-3,4,6,7,9,10-hexahydroacridine-1,8(2H,5H)-dione (exemple 18) ;
- 9-(4-hydroxy-3-méthoxyphényl)-3,3,6,6-tétraméthyl-10-nonyl-3,4,6,7,9,10-hexahydroacridine-1,8(2H,5H)-dione (exemple 19) ;
- 9-(4-hydroxy-3-méthoxyphényl)-3,3,6,6-tétraméthyl-10-undécyl-3,4,6,7,9,10-hexahydroacridine-1,8(2H,5H)-dione (exemple 20) ;
- 10-benzyl-9-(4-hydroxy-3-méthoxyphényl)-3,3,6,6-tétraméthyl-3,4,6,7,9,10-hexahydroacridine-1,8(2H,5H)-dione (exemple 21) ;
- 9-(4-hydroxy-3-méthoxyphényl)-3,3,6,6-tétraméthyl-10-(3-phénylpropyl)-3,4,6,7,9,10-hexahydroacridine-1,8(2H,5H)-dione (exemple 23) ;
- 10-(2,2-diphényléthyl)-9-(4-hydroxy-3-méthoxyphényl)-3,3,6,6-tétraméthyl-3,4,6,7,9,10-hexahydroacridine-1,8(2H,5H)-dione (exemple 24).

**5.** Composé de formule générale (I') :

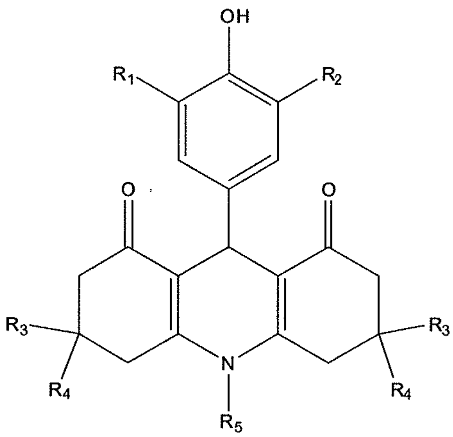

(I')

dans laquelle :

- $R_1$ et $R_2$ représentent simultanément ou indépendamment H, OH, $OCH_3$ ou un radical alkyle en $C_1$-$C_5$,
- $R_3$ et $R_4$ représentent simultanément $CH_3$,
ou $R_3$ représente H et $R_4$ représente $CH_3$, $CH_2CH_3$, un radical isopropyle, ou un radical phényle,
- $R_5$ représente H, un radical alkyle en $C_1$-$C_{24}$, un radical benzyle, un radical phénéthyle ou un radical 3-phénylpropanyle ou 2,2-diphényléthanyle, pour son utilisation topique en tant que médicament.

6. Composé de formule générale (I') selon la revendication 5, **caractérisé en ce qu'**il est choisi parmi l'un des composés suivants :

- 10-dodécyl-9-(4-hydroxy-3-méthoxyphényl)-3,3,6,6-tétraméthyl-3,4,6,7,9,10-hexahydroacridine-1,8(2H,5H)-dione (exemple 1) ;
- 10-décyl-9-(3,4-dihydroxyphényl)-3,3,6,6-tétraméthyl-3,4,6,7,9,10-hexahydroacridine-1,8(2H,5H)-dione (exemple 3) ;
- 9-(3,4-dihydroxyphényl)-10-méthyl-3,6-diphényl-3,4,6,7,9,10-hexahydroacridine-1,8(2H,5H)-dione (exemple 5) ;
- 10-décyl-9-(3,4-dihydroxyphéyl)-3,6-diphéyl-3,4,6,7,9,10-hexahydroacridine-1,8(2H,5H)-dione (exemple 6) ;
- 10-décyl-9-(4-hydroxy-3,5-diméthoxyphényl)-3,3,6,6-tétraméthyl-3,4,6,7,9,10-hexahydroacridine-1,8(2H,5H)-dione (exemple 7) ;
- 10-dodécyl-9-(3,5-di-tert-butyl-4-hydroxyphényl)-3,3,6,6-tétraméthyl-3,4,6,7,9,10-hexahydroacridine-1,8(2H,5H)-dione (exemple 8) ;
- 10-dodécyl-9-(4-hydroxy-3-méthoxyphényi)-3,6-diisopropyl-3,4,6,7,9,10-hexahydroacridine-1,8(2H,5H)-dione (exemple 9) ;
- 10-butyl-9-(4-hydroxy-3-méthoxyphényl)-3,3,6,6-tétraméthyl-3,4,6,7,9,10-hexahydroacridine-1,8(2H,5H)-dione (exemple 14) ;
- 9-(4-hydroxy-3-méthoxyphényl)-3,3,6,6-tétraméthyl-10-pentyl-3,4,6,7,9,10-hexahydroacridine-1,8(2H,5H)-dione (exemple 15) ;
- 10-hexyl-9-(4-hydroxy-3-méthoxyphényl)-3,3,6,6-tétraméthyl-3,4,6,7,9,10-hexahydroacridine-1,8(2H,5H)-dione (exemple 16) ;
- 10-heptyl-9-(4-hydroxy-3-méthoxyphényl)-3,3,6,6-tétraméthyl-3,4,6,7,9,10-hexahydroacridine-1,8(2H,5H)-dione (exemple 17) ;
- 9-(4-hydroxy-3-méthoxyphényl)-3,3,6,6-tétraméthyl-10-octyl-3,4,6,7,9,10-hexahydroacridine-1,8(2H,5H)-dione (exemple 18) ;
- 9-(4-hydroxy-3-méthoxyphényl)-3,3,6,6-tétraméthyl-10-nonyl-3,4,6,7,9,10-hexahydroacridine-1,8(2H,5H)-dione (exemple 19) ;
- 9-(4-hydroxy-3-méthoxyphényl)-3,3,6,6-tétraméthyl-10-undécyl-3,4,6,7,9,10-hexahydroacridine-1,8(2H,5H)-dione (exemple 20) ;
- 10-benzyl-9-(4-hydroxy-3-méthoxyphényl)-3,3,6,6-tétraméthyl-3,4,6,7,9,10-hexahydroacridine-1,8(2H,5H)-dione (exemple 21) ;
- 9-(4-hydroxy-3-méthoxyphényl)-3,3,6,6-tétraméthyl-10-(3-phénylpropyl)-3,4,6,7,9,10-hexahydroacridine-1,8(2H,5H)-dione (exemple 23) ;
- 10-(2,2-diphényléthyl)-9-(4-hydroxy-3-méthoxyphényl)-3,3,6,6-tétraméthyl-3,4,6,7,9,10-hexahydroacridine-1,8(2H,5H)-dione (exemple 24) ;
- 9-(3,4-dihydroxyphényl)-3,3,6,6-tétraméthyl-3,4,6,7,9,10-hexahydroacridine-1,8(2H,5H)-dione (exemple 2) ;
- 9-(3,4-dihydroxyphényl)-3,3,6,6,10-pentaméthyl-3,4,6,7,9,10-hexahydroacridine-1,8(2H,5H)-dione (exemple 4) ;
- 9-(4-hydroxy-3-méthoxyphényl)-3,3,6,6-tétraméthyl-3,4,6,7,9,10-hexahydroacridine-1,8(2H,5H)-dione (exemple 10) ;
- -9-(4-hydroxy-3-méthoxyphényl)-3,3,6,6,10-pentaméthyl-3,4,6,7,9,10-hexahydroacridine-1,8(2H,5H)-dione (exemple 11) ;
- 10-éthyl-9-(4-hydroxy-3-méthoxyphényl)-3,3,6,6-tétraméthyl-3,4,6,7,9,10-hexahydroacridine-1,8(2H,5H)-dione (exemple 12) ;
- 9-(4-hydroxy-3-méthoxyphényl)-3,3,6,6-tétraméthyl-10-propyl-3,4,6,7,9,10-hexahydroacridine-1,8(2H,5H)-dione (exemple 13) ;
- 9-(4-hydroxy-3-méthoxyphényl)-3,3,6,6-tétraméthyl-10-phénéthyl-3,4,6,7,9,10-hexahydroacridine-1,8(2H,5H)-dione (exemple 22).

7. Composé de formule générale (I') selon la revendication 5 pour son utilisation topique dans le traitement et/ou la prévention du vieillissement de la peau.

8. Composé de formule générale (I') selon la revendication 5 pour son utilisation topique visant à modifier la pigmentation de la peau.

**9.** Composé de formule générale (I') selon la revendication 8, **caractérisé en ce que** ledit composé présente un log P > 4 pour son utilisation dans la dépigmentation de la peau et/ou des cheveux et/ou des poils.

**10.** Composé de formule générale (I') selon la revendication 8, **caractérisé en ce que** ledit composé présente un log P < 3,5 pour son utilisation dans la propigmentation de la peau et/ou des cheveux et/ou des poils.

**11.** Utilisation cosmétique d'un composé de formule générale (I') selon la revendication 5 comme agent antioxydant.

**12.** Utilisation cosmétique d'un composé de formule générale (I') selon la revendication 5 comme agent actif dans la pigmentation de la peau.

**13.** Utilisation cosmétique d'un composé de formule générale (I') selon la revendication 12, **caractérisée en ce que** ledit composé présente un log P > 4 comme agent dépigmentant.

**14.** Utilisation cosmétique d'un composé de formule générale (I') selon la revendication 12, **caractérisée en ce que** ledit composé présente un log P < 3,5 comme agent propigmentant.

**15.** Composition topique, **caractérisée en ce qu'**elle comprend à titre de principe actif au moins un composé de formule générale (I') tel que défini selon la revendication 5 en association avec un excipient pharmaceutiquement ou cosmétiquement acceptable.

**16.** Composition topique selon la revendication 15, **caractérisée en ce que** la quantité de composé formule (I') varie entre 0,01 % et 10 % en poids et de préférence de 0,1 % à 5 % en poids par rapport au poids total de la composition.

**17.** Composition topique selon l'une des revendications 15 ou 16, pour son utilisation au traitement et/ou à la prévention du vieillissement de la peau.

**18.** Composition topique selon l'une des revendications 15 ou 16 pour son utilisation visant à modifier la pigmentation de la peau.

**19.** Composition topique selon la revendication 18, **caractérisée en ce que** le composé de formule (I') présente un log P > 4, pour son utilisation dans la dépigmentation de la peau et/ou des cheveux et/ou des poils.

**20.** Composition topique selon la revendication 18, **caractérisée en ce que** le composé de formule (I') présente un log P < 3,5, pour son utilisation dans la propigmentation de la peau et/ou des cheveux et/ou des poils.

**21.** Procédé de préparation des composés de formules générales (I) ou (I') telles que définies à l'une des revendications 1 à 4, **caractérisé en ce qu'**il implique la réaction d'une cyclohexanedione de formule générale (II) :

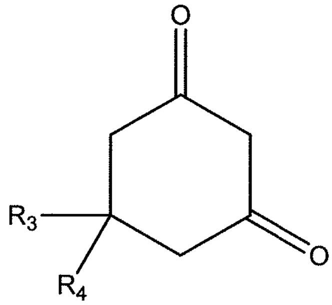

(II)

avec un 4-hydroxybenzaldéhyde de formule générale (III) :

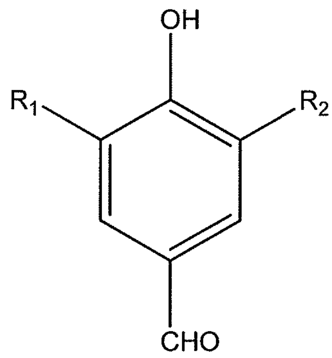

(III)

pour conduire à un produit intermédiaire de formule (IV) :

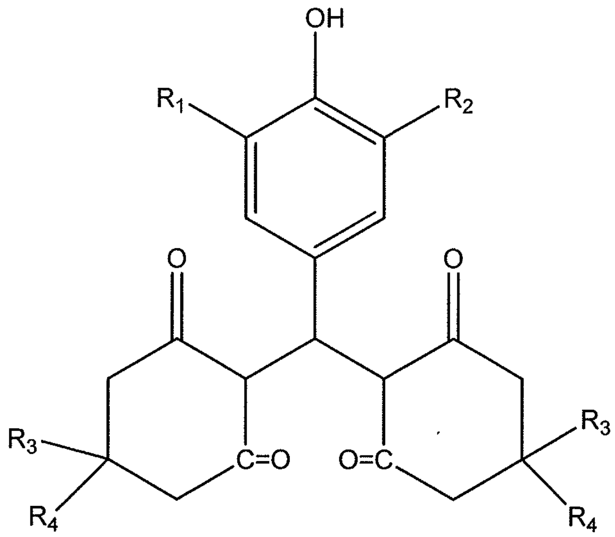

(IV)

qui est mis à réagir avec une alkylamine de formule (V) :

$$R_5NH_2 \qquad (V)$$

pour conduire aux composés de formule (I) correspondants, les significations des radicaux $R_1$ à $R_5$ dans les différentes formules générales (II) à (V) étant telles que définies à l'une des revendications 1 à 6.

**22.** Procédé selon la revendication 21, **caractérisé en ce que** la réaction entre les composés de formules générales (II) et (III) est conduite en présence de pipéridine.

**23.** Procédé selon l'une des revendications 21 et 22, **caractérisé en ce que** la réaction entre les composés de formules générales (IV) et (V) est conduite en présence d'acide acétique.

**Patentansprüche**

**1.** Verbindung mit der allgemeinen Formel (I):

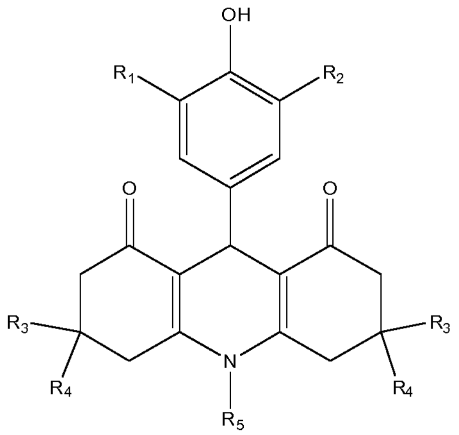

(I)

wobei:

- $R_1$und $R_2$ gleichzeitig und unabhängig H, OH, $OCH_3$ oder ein Alkylradikal mit $C_1$-$C_5$ darstellen,
- $R_3$ und $R_4$ gleichzeitig $CH_3$ darstellen,
- oder $R_3$ gleich H darstellt und $R_4$ gleich $CH_3$, $CH_2CH_3$ oder ein Isopropylradikal oder ein Phenylradikal darstellt,
- $R_5$ ein Alkylradikal mit $C_4$-$C_{24}$ oder ein 3-Phenylpropanyl- oder 2,2-Diphenylethanylradikal darstellt.

2. Verbindung mit der allgemeinen Formel (I) nach Anspruch 1, **dadurch gekennzeichnet, dass** $R_1$gleich H darstellt und $R_2$ gleich $OCH_3$ darstellt.

3. Verbindung mit der allgemeinen Formel (I) nach einem der Ansprüche 1 oder 2, **dadurch gekennzeichnet, dass** $R_3$ und $R_4$ gleichzeitig $CH_3$ darstellen.

4. Verbindung mit der allgemeinen Formel (I) nach einem der Ansprüche 1 bis 3, **dadurch gekennzeichnet, dass** sie ausgewählt ist aus einer der folgenden Verbindungen:

- 10-Dodecyl-9-(4-hydroxy-3-methoxyphenyl)-3,3,6,6-tetramethyl-3,4,6,7,9,10-hexahydroacridin-1,8(2H, 5H)-dion (Beispiel 1);
- 10-Decyl-9-(3,4-dihydroxyphenyl)-3,3,6,6-tetramethyl-3,4,6,7,9,10-hexahydroacridin-1,8 (2H, 5H)-dion (Beispiel 3);
- 9-(3,4-Dihydroxyphenyl)-10-methyl-3,6-diphenyl-3,4,6,7,9,10-hexahydroacridin-1,8(2H,5H)-dion (Beispiel 5)
- 10-Decyl-9-(3,4-dihydroxyphenyl)-3,6-diphenyl-3,4,6,7,9,10-hexahydroacridin-l,8(2H,5H)-dion (Beispiel 6)
- 10-Decyl-9-(4-hydroxy-3,5-dimethoxyphenyl)-3,3,6,6-tetramethyl-3,4,6,7,9,10-hexahydroacridin-1,8(2H, 5H)-dion (Beispiel 7);
- 10-Dodecyl-9-(3,5-di-tert-butyl-4-hydroxyphenyl)-3,3,6,6-tetramethyl-3,4,6,7,9,10-hexahydroacridin-1,8(2H, 5H)-dion (Beispiel 8);
- 10-Dodecyl-9-(4-hydroxy-3-methoxyphenyl)-3,6-diisopropyl-3,4,6,7,9,10-hexahydroacridin-1,8(2H, 5H)-dion (Beispiel 9);
- 10-Butyl-9-(4-hydroxy-3-methoxyphenyl)-3,3,6,6-tetramethyl-3,4,6,7,9,10-hexahydroacridin-1,8(2H, 5H)-dion (Beispiel 14);
- 9-(4-Hydroxy-3-methoxyphenyl)-3,3,6,6-tetramethyl-10-pentyl-3,4,6,7,9,10-hexahydroacridin-1,8(2H,5H)-dion (Beispiel 15);
- 10-Hexyl-9-(4-hydroxy-3-methoxyphenyl)-3,3,6,6-tetramethyl-3,4,6,7,9,10-hexahydroacridin-1,8(2H, 5H)-dion (Beispiel 16);
- 10-Heptyl-9-(4-hydroxy-3-methoxyphenyl)-3,3,6,6-tetramethyl-3,4,6,7,9,10-hexahydroacridin-1,8(2H, 5H)-dion (Beispiel 17);
- 9-(4-Hydroxy-3-methoxyphenyl)-3,3,6,6-tetramethyl-10-octyl-3,4,6,7,9,10-hexahydroacridin-1,8(2H,5H)-dion (Beispiel 18);
- 9-(4-Hydroxy-3-methoxyphenyl)-3,3,6,6-tetramethyl-10-nonyl-3,4,6,7,9,10-hexahydroacridin-l,8(2H,5H)-dion (Beispiel 19);
- 9-(4-Hydroxy-3-methoxyphenyl)-3,3,6,6-tetramethyl-10-undecyl-3,4,6,7,9,10-hexahydroacridin-1,8(2H,5H)-dion (Beispiel 20);

- 10-Benzyl-9-(4-hydroxy-3-methoxyphenyl)-3,3,6,6-tetramethyl-3,4,6,7,9,10-hexahydroacridin-1,8(2H, 5H)-dion (Beispiel 21);
- 9-(4-Hydroxy-3-methoxyphenyl)-3,3,6,6-tetramethyl-10-(3-phenylpropyl)-3,4,6,7,9,10-hexahydroacridin-1,8(2H, 5H)-dion (Beispiel 23);
- 10-(2,2-Diphenylethyl)-9-(4-hydroxy-3-methoxyphenyl)-3,3,6,6-tetramethyl-3,4,6,7,9,10-hexahydroacridin-1,8(2H,5H)-dion (Beispiel 24).

**5.** Verbindung mit der allgemeinen Formel (I'):

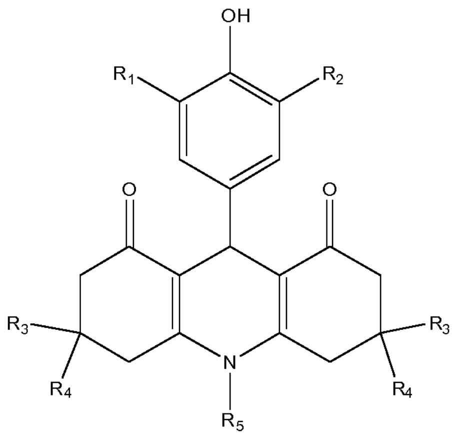

(I)

wobei:

- $R_1$und $R_2$ gleichzeitig und unabhängig H, OH, $OCH_3$ oder ein Alkylradikal mit $C_1$-$C_5$ darstellen,
- $R_3$ und $R_4$ gleichzeitig $CH_3$ darstellen,
oder $R_3$ gleich H darstellt und $R_4$ gleich $CH_3$, $CH_2CH_3$, ein Isopropylradikal oder ein Phenylradikal darstellt,
- $R_5$ gleich H darstellt, ein Alkylradikal mit $C_1$-$C_{24}$, ein Benzylradikal, ein Phenetylradikal oder ein 3-Phenylpropanyl- oder 2,2-Diphenylethanylradikal, für seine topische Verwendung als Medikament.

**6.** Verbindung mit der allgemeinen Formel (I') nach Anspruch 5, **dadurch gekennzeichnet, dass** sie ausgewählt ist aus einer der folgenden Verbindungen:

- 10-Dodecyl-9-(4-hydroxy-3-methoxyphenyl)-3,3,6,6-tetramethyl-3,4,6,7,9,10-hexahydroacridin-1,8(2H, 5H)-dion (Beispiel 1);
- 10-Decyl-9-(3,4-dihydroxyphenyl)-3,3,6,6-tetramethyl-3,4,6,7,9,10-hexahydroacridin-1,8(2H, 5H)-dion (Beispiel 3);
- 9-(3,4-Dihydroxyphenyl)-10-methyl-3,6-diphenyl-3,4,6,7,9,10-hexahydroacridin-1,8(2H,5H)-dion (Beispiel 5);
- 10-Decyl-9-(3,4-dihydroxyphenyl)-3,6-diphenyl-3,4,6,7,9,10-hexahydroacridin-1,8(2H,5H)-dion (Beispiel 6);
- 10-Decyl-9-(4-hydroxy-3,5-dimethoxyphenyl)-3,3,6,6-tetramethyl-3,4,6,7,9,10-hexahydroacridin-1,8(2H, 5H)-dion (Beispiel 7);
- 10-Dodecyl-9-(3,5-di-tert-butyl-4-hydroxyphenyl)-3,3,6,6-tetramethyl-3,4,6,7,9,10-hexahydroacridin-1,8(2H, 5H)-dion (Beispiel 8);
- 10-Dodecyl-9-(4-hydroxy-3-methoxyphenyl)-3,6-diisopropyl-3,4,6,7,9,10-hexahydroacridin-1,8(2H, 5H)-dion (Beispiel 9);
- 10-Butyl-9-(4-hydroxy-3-methoxyphenyl)-3,3,6,6-tetramethyl-3,4,6,7,9,10-hexahydroacridin-1,8(2H, 5H)-dion (Beispiel 14);
- 9-(4-Hydroxy-3-methoxyphenyl)-3,3,6,6-tetramethyl-10-pentyl-3,4,6,7,9,10-hexahydroacridin-1,8(2H,5H)-dion (Beispiel 15);
- 10-Hexyl-9-(4-hydroxy-3-methoxyphenyl)-3,3,6,6-tetramethyl-3,4,6,7,9,10-hexahydroacridin-1,8(2H, 5H)-dion (Beispiel 16);
- 10-Heptyl-9-(4-hydroxy-3-methoxyphenyl)-3,3,6,6-tetramethyl-3,4,6,7,9,10-hexahydroacridin-1,8(2H, 5H)-dion (Beispiel 17);
- 9-(4-Hydroxy-3-methoxyphenyl)-3,3,6,6-tetramethyl-10-octyl-3,4,6,7,9,10-hexahydroacridin-1,8(2H,5H)-dion (Beispiel 18);

- 9-(4-Hydroxy-3-methoxyphenyl)-3,3,6,6-tetramethyl-10-nonyl-3,4,6,7,9,10-hexahydroacridin-1,8(2H,5H)-dion (Beispiel 19);
- 9-(4-Hydroxy-3-methoxyphenyl)-3,3,6,6-tetramethyl-10-undecyl-3,4,6,7,9,10-hexahydroacridin-1,8(2H,5H)-dion (Beispiel 20);
- 10-Benzyl-9-(4-hydroxy-3-methoxyphenyl)-3,3,6,6-tetramethyl-3,4,6,7,9,10-hexahydroacridin-1,8(2H, 5H)-dion (Beispiel 21);
- 9-(4-Hydroxy-3-methoxyphenyl)-3,3,6,6-tetramethyl-10-(3-phenylpropyl)-3,4,6,7,9,10-hexahydroacridin-1,8(2H, 5H)-dion (Beispiel 23);
- 10-(2,2-Diphenylethyl)-9-(4-hydroxy-3-methoxyphenyl)-3,3,6,6-tetramethyl-3,4,6,7,9,10-hexahydroacridin-1,8(2H,5H)-dion (Beispiel 24);
- 9-(3,4-Dihydroxyphenyl)-3,3,6,6-tetramethyl-3,4,6,7,9,10-hexahydroacridin-1,8(2H,5H)-dion (Beispiel 2);
- 9-(3,4-Dihydroxyphenyl)-3,3,6,6,10-pentamethyl-3,4,6,7,9,10-hexahydroacridin-1,8(2H,5H)-dion (Beispiel 4);
- 9-(4-Hydroxy-3-methoxyphenyl)-3,3,6,6-tetramethyl-3,4,6,7,9,10-hexahydroacridin-1,8(2H,5H)-dion (Beispiel 10);
- -9-(4-Hydroxy-3-methoxyphenyl)-3,3,6,6,10-pentamethyl-3,4,6,7,9,10-hexahydroacridin-1,8(2H, 5H)-dion (Beispiel 11);
- 10-Ethyl-9-(4-hydroxy-3-methoxyphenyl)-3,3,6,6-tetramethyl-3,4,6,7,9,10-hexahydroacridin-1,8(2H, 5H)-dion (Beispiel 12);
- 9-(4-Hydroxy-3-methoxyphenyl)-3,3,6,6-tetramethyl-10-propyl-3,4,6,7,9,10-hexahydroacridin-1,8(2H,5H)-dion (Beispiel 13);
- 9-(4-Hydroxy-3-methoxyphenyl)-3,3,6,6-tetramethyl-10-phenethyl-3,4,6,7,9,10-hexahydroacridin-1,8(2H, 5H)-dion (Beispiel 22).

**7.** Verbindung mit der allgemeinen Formel (I') nach Anspruch 5 für ihre topische Verwendung bei der Behandlung und/oder der Prävention der Alterung der Haut.

**8.** Verbindung mit der allgemeinen Formel (I') nach Anspruch 5 für ihre topische Verwendung, um die Pigmentierung der Haut zu verbessern.

**9.** Verbindung mit der allgemeinen Formel (I) nach Anspruch 8, **dadurch gekennzeichnet, dass** die Verbindung einen Log P > 4 aufweist, für seine Verwendung bei der Entpigmentierung der Haut und/oder der Haare und/oder der Körperhaare.

**10.** Verbindung mit der allgemeinen Formel (I') nach Anspruch 8, **dadurch gekennzeichnet, dass** die Verbindung einen Log P < 3,5 aufweist, für ihre Verwendung bei der Propigmentierung der Haut und/oder der Haare und/oder der Körperhaare.

**11.** Kosmetische Verbindung mit der allgemeinen Formel (I') nach Anspruch 5 als Antioxidationsmittel.

**12.** Kosmetische Verbindung mit der allgemeinen Formel (I') nach Anspruch 5 als Wirkstoff bei der Pigmentierung der Haut.

**13.** Verwendung einer kosmetischen Verbindung mit der allgemeinen Formel (I') nach Anspruch 12, **dadurch gekennzeichnet, dass** die Verbindung einen Log P > 4 als Entpigmentierungsmittel aufweist.

**14.** Kosmetische Verwendung einer Verbindung mit der allgemeinen Formel (I') nach Anspruch 12, **dadurch gekennzeichnet, dass** die Verbindung einen Log P < 3,5 als Propigmentierungsmittel aufweist.

**15.** Topische Zusammensetzung, **dadurch gekennzeichnet, dass** sie als Wirkstoff mindestens eine Verbindung mit der allgemeinen Formel (I') umfasst, wie nach Anspruch 5 definiert, in Verbindung mit einem pharmazeutisch oder kosmetisch annehmbaren Hilfsstoff.

**16.** Zusammensetzung nach Anspruch 15, **dadurch gekennzeichnet, dass** die Menge der Verbindung mit der Formel (I') zwischen 0,01 Gew% und 10 Gew% und vorzugsweise von 0,1 Gew% bis 5 Gew% mit Bezug auf das Gesamtgewicht der Zusammensetzung variiert.

**17.** Zusammensetzung nach einem der Ansprüche 15 oder 16 für ihre topische Verwendung bei der Behandlung und/oder der Prävention der Alterung der Haut.

**18.** Topische Zusammensetzung nach einem der Ansprüche 15 oder 16 für ihre topische Verwendung, um die Pigmentierung der Haut zu verbessern.

**19.** Topische Zusammensetzung nach Anspruch 18, **dadurch gekennzeichnet, dass** die Verbindung mit der Formel (I') einen Log P > 4 aufweist, für ihre Verwendung bei der Entpigmentierung der Haut und/oder der Haare und/oder der Körperhaare.

**20.** Topische Zusammensetzung nach Anspruch 18, **dadurch gekennzeichnet, dass** die Verbindung mit der Formel (I') einen Log P < 3,5 aufweist, für ihre Verwendung bei der Propigmentierung der Haut und/oder der Haare und/oder der Körperhaare.

**21.** Verfahren zur Herstellung der Verbindungen der allgemeinen Formeln (I) oder (I'), wie in einem der Ansprüche 1 bis 4 definiert, **dadurch gekennzeichnet, dass** es die Reaktion eines Cyclohexadions mit der folgenden allgemeinen Formel (II) impliziert:

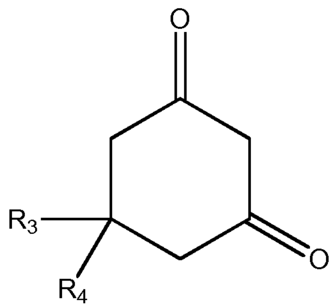

(II)

mit einem 4-Hydroxybenzaldehyd mit der allgemeinen Formel (III) :

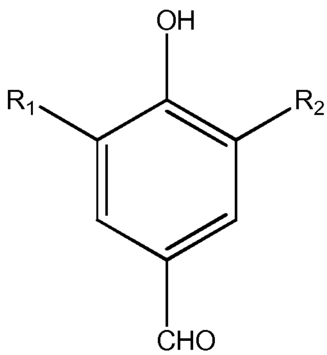

(III)

um zu einem Zwischenprodukt mit der folgenden Formel (IV) zu führen:

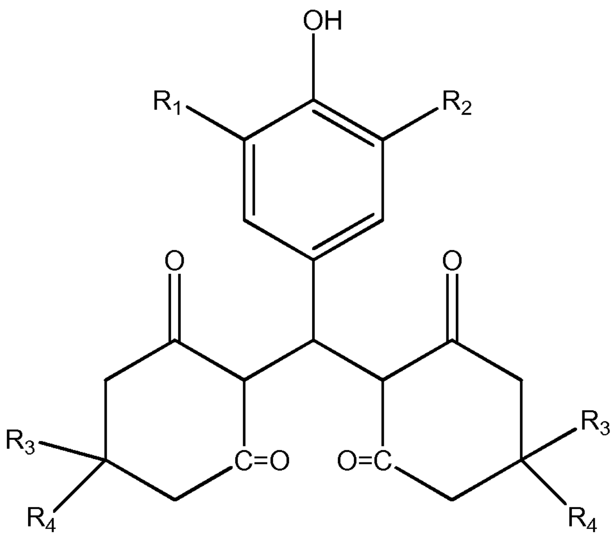

(IV)

die mit einem Alkylamin mit der Formel (V) zur Reaktion gebracht wird:

$R_5NH_2$ (V).

um zu den entsprechenden Verbindungen mit der Formel (I) zu führen, wobei die Bedeutungen der Radikale $R_1$ bis $R_5$ in den verschiedenen allgemeinen Formeln (II) bis (V) derart sind, wie in einem der Ansprüche 1 bis 6 definiert.

**22.** Verfahren nach Anspruch 21, **dadurch gekennzeichnet, dass** die Reaktion zwischen den Verbindungen mit den allgemeinen Formeln (II) und (III) in Anwesenheit von Piperidin durchgeführt wird.

**23.** Verfahren nach einem der Ansprüche 21 und 22, **dadurch gekennzeichnet, dass** die Reaktion zwischen den Verbindungen mit den allgemeinen Formeln (IV) und (V) in Anwesenheit von Essigsäure durchgeführt wird.

## Claims

**1.** Compound of general formula (I):

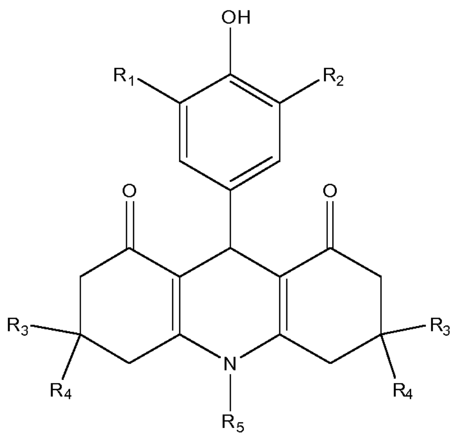

(I)

in which:

- $R_1$ and $R_2$ represent simultaneously or independently H, OH, $OCH_3$ or a $C_1$-$C_5$ alkyl radical,
- $R_3$ and $R_4$ represent simultaneously $CH_3$,
- or $R_3$ represents H and $R_4$ represents $CH_3$, $CH_2CH_3$ or an isopropyl radical or a phenyl radical,
- $R_5$ represents a $C_4$-$C_{24}$ alkyl radical or a 3-phenylpropanyl or 2,2-diphenylethanyl radical.

**2.** Compound of general formula (I) according to claim 1, **characterised in that** $R_1$represents H and $R_2$ represents $OCH_3$.

**3.** Compound of general formula (I) according to one of claims 1 or 2, **characterised in that** $R_3$ and $R_4$ represent simultaneously $CH_3$.

**4.** Compound of general formula (I) according to one of claims 1 to 3, **characterised in that** it is chosen from one of the following compounds:

- 10-dodecyl-9-(4-hydroxy-3-methoxyphenyl)-3,3,6,6-tetramethyl-3,4,6,7,9,10-hexahydroacridine-1,8(2H,5H)-dione (example 1);
- 10-decyl-9-(3,4-dihydroxyphenyl)-3,3,6,6-tetramethyl-3,4,6,7,9,10-hexahydroacridine-1,8(2H,5H)-dione (example 3) ;
- 9-(3,4-dihydroxyphenyl)-10-methyl-3,6-diphenyl-3,4,6,7,9,10-hexahydroacridine-l,8(2H,5H)-dione (example 5)
- 10-decyl-9-(3,4-dihydroxypheyl)-3,6-dipheyl-3,4,6,7,9,10-hexahydroacridine-1,8(2H,5H)-dione (example 6)
- 10-decyl-9-(4-hydroxy-3,5-dimethoxyphenyl)-3,3,6,6-tetramethyl-3,4,6,7,9,10-hexahydroacridine-1,8(2H,5H)-dione (example 7);
- 10-dodecyl-9-(3,5-di-tert-butyl-4-hydroxyphenyl)-3,3,6,6-tetramethyl-3,4,6,7,9,10-hexahydroacridine-1,8(2H,5H)-dione (example 8);
- 10-dodecyl-9-(4-hydroxy-3-methoxyphenyl)-3,6-diisopropyl-3,4,6,7,9,10-hexahydroacridine-1,8(2H,5H)-dione (example 9);
- 10-butyl-9-(4-hydroxy-3-methoxyphenyl)-3,3,6,6-tetramethyl-3,4,6,7,9,10-hexahydroacridine-1,8(2H,5H)-dione (example 14);
- 9-(4-hydroxy-3-methoxyphenyl)-3,3,6,6-tetramethyl-10-pentyl-3,4,6,7,9,10-hexahydroacridine-1,8(2H,5H)-dione (example 15);
- 10-hexyl-9-(4-hydroxy-3-methoxyphenyl)-3,3,6,6-tetramethyl-3,4,6,7,9,10-hexahydroacridine-1,8(2H,5H)-dione (example 16);
- 10-heptyl-9-(4-hydroxy-3-methoxyphenyl)-3,3,6,6-tetramethyl-3,4,6,7,9,10-hexahydroacridine-1,8(2H,5H)-dione (example 17);
- 9-(4-hydroxy-3-methoxyphenyl)-3,3,6,6-tetramethyl-10-octyl-3,4,6,7,9,10-hexahydroacridine-1,8(2H,5H)-dione (example 18);
- 9-(4-hydroxy-3-methoxyphenyl)-3,3,6,6-tetramethyl-10-nonyl-3,4,6,7,9,10-hexahydroacridine-1,8(2H,5H)-dione (example 19);
- 9-(4-hydroxy-3-methoxyphenyl)-3,3,6,6-tetramethyl-10-undecyl-3,4,6,7,9,10-hexahydroacridine-1,8(2H,5H)-dione (example 20);
- 10-benzyl-9-(4-hydroxy-3-methoxyphenyl)-3,3,6,6-tetramethyl-3,4,6,7,9,10-hexahydroacridine-1,8(2H,5H)-dione (example 21);
- 9-(4-hydroxy-3-methoxyphenyl)-3,3,6,6-tetramethyl-10-(3-phenylpropyl)-3,4,6,7,9,10-hexahydroacridine-1,8(2H,5H)-dione (example 23);
- 10-(2,2-diphenylethyl)-9-(4-hydroxy-3-methoxyphenyl)-3,3,6,6-tetramethyl-3,4,6,7,9,10-hexahydroacridine-1,8(2H,5H)-dione (example 24).

**5.** Compound of general formula (I'):

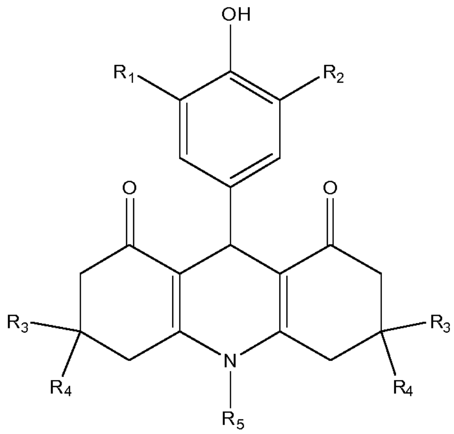

(I')

in which:

- $R_1$ and $R_2$ represent simultaneously or independently H, OH, $OCH_3$ or a $C_1$-$C_5$ alkyl radical,
- $R_3$ and $R_4$ represent simultaneously $CH_3$, or $R_3$ represents H and $R_4$ represents $CH_3$, $CH_2CH_3$, an isopropyl radical or a phenyl radical,
- $R_5$ represents H, a $C_1$-$C_{24}$ alkyl radical, a benzyl radical, a phenethyl radical or a 3-phenylpropanyl or 2,2-diphenylethanyl radical,

for topical use thereof as a medication.

**6.** Compound of general formula (I') according to claim 5, **characterised in that** it is chosen from one of the following compounds:

- 10-dodecyl-9-(4-hydroxy-3-methoxyphenyl)-3,3,6,6-tetramethyl-3,4,6,7,9,10-hexahydroacridine-1,8(2H,5H)-dione (example 1);
- 10-decyl-9-(3,4-dihydroxyphenyl)-3,3,6,6-tetramethyl-3,4,6,7,9,10-hexahydroacridine-1,8(2H,5H)-dione (example 3) ;
- 9-(3,4-dihydroxyphenyl)-10-methyl-3,6-diphenyl-3,4,6,7,9,10-hexahydroacridine-1,8(2H,5H)-dione (example 5) ;
- 10-decyl-9-(3,4-dihydroxypheyl)-3,6-dipheyl-3,4,6,7,9,10-hexahydroacridine-1,8(2H,5H)-dione (example 6) ;
- 10-decyl-9-(4-hydroxy-3,5-dimethoxyphenyl)-3,3,6,6-tetramethyl-3,4,6,7,9,10-hexahydroacridine-1,8(2H,5H)-dione (example 7);
- 10-dodecyl-9-(3,5-di-tert-butyl-4-hydroxyphenyl)-3,3,6,6-tetramethyl-3,4,6,7,9,10-hexahydroacridine-1,8(2H,5H)-dione (example 8);
- 10-dodecyl-9-(4-hydroxy-3-methoxyphenyl)-3,6-diisopropyl-3,4,6,7,9,10-hexahydroacridine-1,8(2H,5H)-dione (example 9);
- 10-butyl-9-(4-hydroxy-3-methoxyphenyl)-3,3,6,6-tetramethyl-3,4,6,7,9,10-hexahydroacridine-1,8(2H,5H)-dione (example 14);
- 9-(4-hydroxy-3-methoxyphenyl)-3,3,6,6-tetramethyl-10-pentyl-3,4,6,7,9,10-hexahydroacridine-1,8(2H,5H)-dione (example 15);
- 10-hexyl-9-(4-hydroxy-3-methoxyphenyl)-3,3,6,6-tetramethyl-3,4,6,7,9,10-hexahydroacridine-1,8(2H,5H)-dione (example 16);
- 10-heptyl-9-(4-hydroxy-3-methoxyphenyl)-3,3,6,6-tetramethyl-3,4,6,7,9,10-hexahydroacridine-1,8(2H,5H)-dione (example 17);
- 9-(4-hydroxy-3-methoxyphenyl)-3,3,6,6-tetramethyl-10-octyl-3,4,6,7,9,10-hexahydroacridine-1,8(2H,5H)-dione (example 18);
- 9-(4-hydroxy-3-methoxyphenyl)-3,3,6,6-tetramethyl-10-nonyl-3,4,6,7,9,10-hexahydroacridine-1,8(2H,5H)-dione (example 19);
- 9-(4-hydroxy-3-methoxyphenyl)-3,3,6,6-tetramethyl-10-undecyl-3,4,6,7,9,10-hexahydroacridine-1,8(2H,5H)-dione (example 20);
- 10-benzyl-9-(4-hydroxy-3-methoxyphenyl)-3,3,6,6-tetramethyl-3,4,6,7,9,10-hexahydroacridine-1,8(2H,5H)-dione (example 21);

- 9-(4-hydroxy-3-methoxyphenyl)-3,3,6,6-tetramethyl-10-(3-phenylpropyl)-3,4,6,7,9,10-hexahydroacridine-1,8(2H,5H)-dione (example 23);
- 10-(2,2-diphenylethyl)-9-(4-hydroxy-3-methoxyphenyl)-3,3,6,6-tetramethyl-3,4,6,7,9,10-hexahydroacridine-1,8(2H,5H)-dione (example 24);
- 9-(3,4-dihydroxyphenyl)-3,3,6,6-tetramethyl-3,4,6,7,9,10-hexahydroacridine-1,8(2H,5H)-dione (example 2) ;
- 9-(3,4-dihydroxyphenyl)-3,3,6,6,10-pentamethyl-3,4,6,7,9,10-hexahydroacridine-1,8(2H,5H)-dione (example 4) ;
- 9-(4-hydroxy-3-methoxyphenyl)-3,3,6,6-tetramethyl-3,4,6,7,9,10-hexahydroacridine-1,8(2H,5H)-dione (example 10) ;
- -9-(4-hydroxy-3-methoxyphenyl)-3,3,6,6,10-pentamethyl-3,4,6,7,9,10-hexahydroacridine-1,8(2H,5H)-dione (example 11) ;
- 10-ethyl-9-(4-hydroxy-3-methoxyphenyl)-3,3,6,6-tetramethyl-3,4,6,7,9,10-hexahydroacridine-1,8(2H,5H)-dione (example 12);
- 9-(4-hydroxy-3-methoxyphenyl)-3,3,6,6-tetramethyl-10-propyl-3,4,6,7,9,10-hexahydroacridine-1,8(2H,5H)-dione (example 13);
- 9-(4-hydroxy-3-methoxyphenyl)-3,3,6,6-tetramethyl-10-phenethyl-3,4,6,7,9,10-hexahydroacridine-1,8(2H,5H)-dione (example 22).

**7.** Compound of general formula (I') according to claim 5, for topical use thereof in the treatment and/or prevention of skin ageing.

**8.** Compound of general formula (I') according to claim 5, for topical use thereof aimed at modifying skin pigmentation.

**9.** Compound of general formula (I') according to claim 8, **characterised in that** said compound has a log P > 4 for use thereof in depigmentation of the skin and/or head and/or facial and/or body hair.

**10.** Compound of general formula (I') according to claim 8, **characterised in that** said compound has a log P < 3.5 for use thereof in propigmentation of the skin and/or head and/or facial and/or body hair.

**11.** Cosmetic use of a compound of general formula (I') according to claim 5 as an antioxidant.

**12.** Cosmetic use of a compound of general formula (I') according to claim 5 as an active agent in skin pigmentation.

**13.** Cosmetic use of a compound of general formula (I') according to claim 12, **characterised in that** said compound has a log P > 4 as a depigmentation agent.

**14.** Cosmetic use of a compound of general formula (I') according to claim 12, **characterised in that** said compound has a log P < 3.5 as a propigmentation agent.

**15.** Topical composition, **characterised in that** it comprises, by way of active principle, at least one compound of general formula (I') as defined according to claim 5 in association with a pharmaceutically or cosmetically acceptable excipient.

**16.** Topical composition according to claim 15, **characterised in that** the quantity of compound of formula (I') varies between 0.01% and 10% by weight and preferably 0.1% to 5% by weight with respect to the total weight of the composition.

**17.** Topical composition according to one of claims 15 or 16, for use thereof in the treatment and/or prevention of skin ageing.

**18.** Topical composition according to one of claims 15 or 16 for use thereof aimed at modifying skin pigmentation.

**19.** Topical composition according to claim 18, **characterised in that** the compound of formula (I') has a log P > 4, for use thereof in depigmentation of the skin and/or head and/or facial and/or body hair.

**20.** Topical composition according to claim 18, **characterised in that** the compound of formula (I') has a log P < 3.5, for use thereof in propigmentation of the skin and/or head and/or facial and/or body hair.

**21.** Method for preparing compounds of general formulae (I) or (I') as defined in one of claims 1 to 4, **characterised in**

**that** it involves the reaction of a cyclohexanedione of general formula (II):

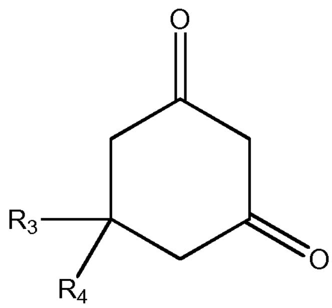

(II)

with a 4-hydroxybenzaldehyde of general formula (III):

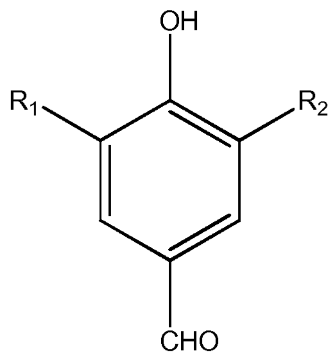

(III)

for leading to an intermediate product of formula (IV):

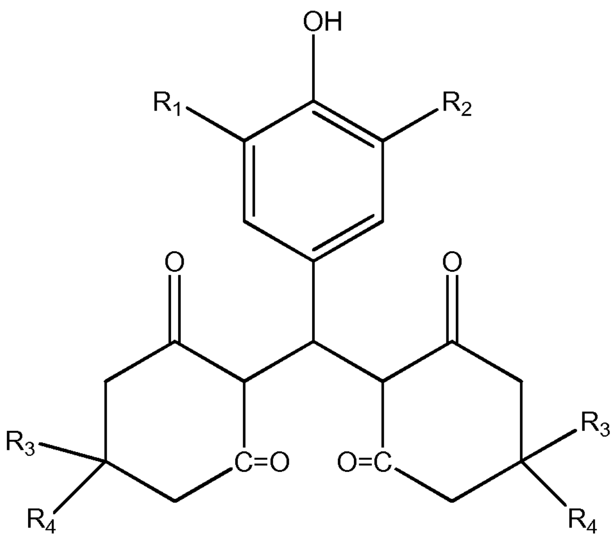

(IV)

which is reacted with an alkylamine of formula (V):

$R_5NH_2$ (V)

to lead to the corresponding compounds of formula (I), the meanings of the radicals $R_1$ to $R_5$ in the various general formulae (II) to (V) being as defined in one of claims 1 to 6.

**22.** Method according to claim 21, **characterised in that** the reaction between the compounds of general formulae (II) and (III) is carried out in the presence of piperidine.

**23.** Method according to one of claims 21 and 22, **characterised in that** the reaction between the compounds of general formulae (IV) and (V) is carried out in the presence of acetic acid.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- Microwave assisted one-pot synthesis of highly potent novel isoniazid analogues. **THIMMAPPA H. MANJASHETTY et al.** Bioorg. & Med. Chem. Lett. Pergamon, Elsevier Science, 28 Janvier 2011, vol. 21, 2125-28
- **C. HANSCH ; J. F. QUINLAN ; G. L. LAWRENCE.** Linear free energy relationship between partition coefficients and the aqueous solubility of organic liquids. *J. Org. Chem.,* 1968, vol. 33, 347-350
- **C. HANSCH ; A. LEO.** Exploring QSAR - Fundamentals and Applications in Chemistry and Biology. American Chemical Society, 1995
- **J. SANGSTER.** Octanol - Water Partition Coefficients: Fundamentals and Physical Chemistry. Wiley, 1997
- **W. JORGENSEN ; E. DUFFY.** *Advanced Drug Delivery Reviews,* 2002, 355-366